# EUROPEAN PATENT SPECIFICATION

(11) **EP 2 850 207 B1**
(45) Date of publication and mention of the grant of the patent: **04.01.2017**
(21) Application number: 13725930.5
(22) Date of filing: 15.05.2013
(51) Int. Cl.: C12Q 1/68

(54) **METHODS FOR THE DIAGNOSIS AND THE TREATMENT OF FAMILIAL THORACIC AORTIC ANEURYSMS CAUSED BY TGFB2 LOSS OF FUNCTION MUTATIONS**
VERFAHREN ZUR DIAGNOSE UND BEHANDLUNG VON FAMILIÄREN ANEURYSMEN DER A. THORACICA DURCH TGFB2-FUNKTIONSVERLUSTMUTATIONEN
PROCÉDÉS DE DIAGNOSTIC ET DE TRAITEMENT D'ANÉVRISMES AORTIQUES THORACIQUES FAMILIAUX PROVOQUÉS PAR DES MUTATIONS PERTE DE FONCTION DU GÈNE TGFB2

(30) Priority: 16.05.2012 EP 12305549
(43) Date of publication of application: 25.03.2015
(73) Proprietor: Institut National de la Santé et de la Recherche Médicale (INSERM), 75013 Paris (FR); Université Paris Diderot - Paris 7, 75205 Paris Cedex 13 (FR); Université de Versailles Saint-Quentin-en-Yvelines, 78035 Versailles Cédex (FR); UNIVERSITE PARIS 13 PARIS NORD, 93430 Villetaneuse (FR); Assistance Publique Hôpitaux de Paris, 75004 Paris (FR); Board of Regents of the University of Texas System, Austin, TX 78701 (US)
(72) Inventor: BOILEAU, Catherine, F-75877 Paris Cedex 18 (FR); MILEWICZ, Dianna, Houston, Texas 77030 (US); JONDEAU, Guillaume, F-75877 Paris Cedex 18 (FR)
(74) Representative: Nony
(86) International application number: PCT/EP2013/059993
(87) International publication number: WO 2013/171244

(56) References cited:
- WO-A1-2009/056419
- WO-A1-2011/136638
- WANG YU ET AL: "TGF-beta activity protects against inflammatory aortic aneurysm progression and complications in angiotensin II-infused mice", JOURNAL OF CLINICAL INVESTIGATION, AMERICAN SOCIETY FOR CLINICAL INVESTIGATION, US, vol. 120, no. 2, 1 February 2010 (2010-02-01), pages 422-432, XP009162240, ISSN: 0021-9738
- MARK E. LINDSAY ET AL: "Lessons on the pathogenesis of aneurysm from heritable conditions", NATURE, vol. 473, no. 7347, 18 May 2011 (2011-05-18), pages 308-316, XP055077047, ISSN: 0028-0836, DOI: 10.1038/nature10145
- SIDDHARTH K. PRAKASH ET AL: "Rare Copy Number Variants Disrupt Genes Regulating Vascular Smooth Muscle Cell Adhesion and Contractility in Sporadic Thoracic Aortic Aneurysms and Dissections", THE AMERICAN JOURNAL OF HUMAN GENETICS, vol. 87, no. 6, 1 December 2010 (2010-12-01), pages 743-756, XP055076337, ISSN: 0002-9297, DOI: 10.1016/j.ajhg.2010.09.015 cited in the application
- BART L. LOEYS ET AL: "Aneurysm Syndromes Caused by Mutations in the TGF-[beta] Receptor", NEW ENGLAND JOURNAL OF MEDICINE, vol. 355, no. 8, 24 August 2006 (2006-08-24), pages 788-798, XP055076334, ISSN: 0028-4793, DOI: 10.1056/NEJMoa055695 cited in the application
- BOILEAU CATHERINE ET AL: "Molecular genetics of Marfan syndrome", CURRENT OPINION IN CARDIOLOGY, GOWER ACADEMIC JOURNALS, LONDON, GB, vol. 20, no. 3, 1 May 2005 (2005-05-01), pages 194-200, XP009120592, ISSN: 0268-4705, DOI: 10.1097/01.HCO.0000162398.21972.CD
- CATHERINE BOILEAU ET AL: "TGFB2 mutations cause familial thoracic aortic aneurysms and dissections associated with mild systemic features of Marfan syndrome", NATURE GENETICS, vol. 44, no. 8, 8 July 2012 (2012-07-08), pages 916-921, XP055076847, ISSN: 1061-4036, DOI: 10.1038/ng.2348
- MARK E LINDSAY ET AL: "Loss-of-function mutations in TGFB2 cause a syndromic presentation of thoracic aortic aneurysm", NATURE GENETICS, vol. 44, no. 8, 8 July 2012 (2012-07-08), pages 922-927, XP055077055, ISSN: 1061-4036, DOI: 10.1038/ng.2349

### SEQUENCE LISTING

<110> INSERM
<120> METHODS FOR THE DIAGNOSIS AND THE TREATMENT OF FAMILIAL THORACIC AORTIC ANEURYSMS CAUSED BY TGFB2 LOSS OF FUNCTION MUTATIONS
<130> BI012150 BOILEAU / MC
<160> 17
<170> PatentIn version 3.3
<210> 1
   <211> 5882
   <212> DNA
   <213> Homo sapiens
<400> 1
   gtgatgttat ctgctggcag cagaaggttc gctccgagcg gagctccaga agctcctgac     60
   aagagaaaga cagattgaga tagagataga aagagaaaga gagaaagaga cagcagagcg    120
   agagcgcaag tgaaagaggc aggggagggg gatggagaat attagcctga cggtctaggg    180
   agtcatccag gaacaaactg aggggctgcc cggctgcaga caggaggaga cagagaggat    240
   ctattttagg gtggcaagtg cctacctacc ctaagcgagc aattccacgt tggggagaag    300
   ccagcagagg ttgggaaagg gtgggagtcc aagggagccc ctgcgcaacc ccctcaggaa    360
   taaaactccc cagccagggt gtcgcaaggg ctgccgttgt gatccgcagg gggtgaacgc    420
   aaccgcgacg gctgatcgtc tgtggctggg ttggcgtttg gagcaagaga aggaggagca    480
   ggagaaggag ggagctggag gctggaagcg tttgcaagcg gcggcggcag caacgtggag    540
   taaccaagcg ggtcagcgcg cgcccgccag ggtgtaggcc acggagcgca gctcccagag    600
   caggatccgc gccgcctcag cagcctctgc ggcccctgcg gcacccgacc gagtaccgag    660
   cgccctgcga agcgcaccct cctccccgcg gtgcgctggg ctcgccccca gcgcgcgcac    720
   acgcacacac acacacacac acacacacgc acgcacacac gtgtgcgctt ctctgctccg    780
   gagctgctgc tgctcctgct ctcagcgccg cagtggaagg caggaccgaa ccgctccttc    840
   tttaaatata taaatttcag cccaggtcag cctcggcggc ccccctcacc gcgctcccgg    900
   cgcccctccc gtcagttcgc cagctgccag ccccgggacc ttttcatctc ttcccttttg    960
   gccggaggag ccgagttcag atccgccact ccgcacccga gactgacaca ctgaactcca   1020
   cttcctcctc ttaaatttat ttctacttaa tagccactcg tctctttttt tccccatctc   1080
   attgctccaa gaattttttt cttcttactc gccaaagtca gggttccctc tgcccgtccc   1140
   gtattaatat ttccactttt ggaactactg gccttttctt tttaaaggaa ttcaagcagg   1200
   atacgttttt ctgttgggca ttgactagat tgtttgcaaa agtttcgcat caaaaacaac   1260
   aacaacaaaa aaccaaacaa ctctccttga tctatacttt gagaattgtt gatttctttt   1320
   ttttattctg acttttaaaa acaacttttt tttccacttt tttaaaaaat gcactactgt 1380
   gtgctgagcg cttttctgat cctgcatctg gtcacggtcg cgctcagcct gtctacctgc 1440
   agcacactcg atatggacca gttcatgcgc aagaggatcg aggcgatccg cgggcagatc 1500
   ctgagcaagc tgaagctcac cagtccccca gaagactatc ctgagcccga ggaagtcccc 1560
   ccggaggtga tttccatcta caacagcacc agggacttgc tccaggagaa ggcgagccgg 1620
   agggcggccg cctgcgagcg cgagaggagc gacgaagagt actacgccaa ggaggtttac 1680
   aaaatagaca tgccgccctt cttcccctcc gaaaatgcca tcccgcccac tttctacaga 1740
   ccctacttca gaattgttcg atttgacgtc tcagcaatgg agaagaatgc ttccaatttg 1800
   gtgaaagcag agttcagagt ctttcgtttg cagaacccaa aagccagagt gcctgaacaa 1860
   cggattgagc tatatcagat tctcaagtcc aaagatttaa catctccaac ccagcgctac 1920
   atcgacagca aagttgtgaa aacaagagca gaaggcgaat ggctctcctt cgatgtaact 1980
   gatgctgttc atgaatggct tcaccataaa gacaggaacc tgggatttaa aataagctta 2040
   cactgtccct gctgcacttt tgtaccatct aataattaca tcatcccaaa taaaagtgaa 2100
   gaactagaag caagatttgc aggtattgat ggcacctcca catataccag tggtgatcag 2160
   aaaactataa agtccactag gaaaaaaaac agtgggaaga ccccacatct cctgctaatg 2220
   ttattgccct cctacagact tgagtcacaa cagaccaacc ggcggaagaa gcgtgctttg 2280
   gatgcggcct attgctttag aaatgtgcag gataattgct gcctacgtcc actttacatt 2340
   gatttcaaga gggatctagg gtggaaatgg atacacgaac ccaaagggta caatgccaac 2400
   ttctgtgctg gagcatgccc gtatttatgg agttcagaca ctcagcacag cagggtcctg 2460
   agcttatata ataccataaa tccagaagca tctgcttctc cttgctgcgt gtcccaagat 2520
   ttagaacctc taaccattct ctactacatt ggcaaaacac ccaagattga acagctttct 2580
   aatatgattg taaagtcttg caaatgcagc taaaattctt ggaaaagtgg caagaccaaa 2640
   atgacaatga tgatgataat gatgatgacg acgacaacga tgatgcttgt aacaagaaaa 2700
   cataagagag ccttggttca tcagtgttaa aaaatttttg aaaaggcggt actagttcag 2760
   acactttgga agtttgtgtt ctgtttgtta aaactggcat ctgacacaaa aaaagttgaa 2820
   ggccttattc tacatttcac ctactttgta agtgagagag acaagaagca aatttttttt 2880
   aaagaaaaaa ataaacactg gaagaattta ttagtgttaa ttatgtgaac aacgacaaca 2940
   acaacaacaa caacaaacag gaaaatccca ttaagtggag ttgctgtacg taccgttcct 3000
   atcccgcgcc tcacttgatt tttctgtatt gctatgcaat aggcacccttcccattctta 3060
   ctcttagagt taacagtgag ttatttattg tgtgttacta tataatgaac gtttcattgc 3120
   ccttggaaaa taaaacaggt gtataaagtg gagaccaaat actttgccag aaactcatgg 3180
   atggcttaag gaacttgaac tcaaacgagc cagaaaaaaa gaggtcatat taatgggatg   3240
   aaaacccaag tgagttatta tatgaccgag aaagtctgca ttaagataaa gaccctgaaa 3300
   acacatgtta tgtatcagct gcctaaggaa gcttcttgta aggtccaaaa actaaaaaga 3360
   ctgttaataa aagaaacttt cagtcagaat aagtctgtaa gttttttttt ttctttttaa 3420
   ttgtaaatgg ttctttgtca gtttagtaaa ccagtgaaat gttgaaatgt tttgacatgt 3480
   actggtcaaa cttcagacct taaaatattg ctgtatagct atgctatagg ttttttcctt 3540
   tgttttggta tatgtaacca tacctatatt attaaaatag atggatatag aagccagcat 3600
   aattgaaaac acatctgcag atctcttttg caaactatta aatcaaaaca ttaactactt 3660
   tatgtgtaat gtgtaaattt ttaccatatt ttttatattc tgtaataatg tcaactatga 3720
   tttagattga cttaaatttg ggctcttttt aatgatcact cacaaatgta tgtttctttt 3780
   agctggccag tacttttgag taaagcccct atagtttgac ttgcactaca aatgcatttt 3840
   ttttttaata acatttgccc tacttgtgct ttgtgtttct ttcattatta tgacataagc 3900
   tacctgggtc cacttgtctt ttcttttttt tgtttcacag aaaagatggg ttcgagttca 3960
   gtggtcttca tcttccaagc atcattacta accaagtcag acgttaacaa atttttatgt 4020
   taggaaaagg aggaatgtta tagatacata gaaaattgaa gtaaaatgtt ttcattttag 4080
   caaggattta gggttctaac taaaactcag aatctttatt gagttaagaa aagtttctct 4140
   accttggttt aatcaatatt tttgtaaaat cctattgtta ttacaaagag gacacttcat 4200
   aggaaacatc tttttcttta gtcaggtttt taatattcag ggggaaattg aaagatatat 4260
   attttagtcg atttttcaaa aggggaaaaa agtccaggtc agcataagtc attttgtgta 4320
   tttcactgaa gttataaggt ttttataaat gttctttgaa ggggaaaagg cacaagccaa 4380
   tttttcctat gatcaaaaaa ttctttcttt cctctgagtg agagttatct atatctgagg 4440
   ctaaagttta ccttgcttta ataaataatt tgccacatca ttgcagaaga ggtatcctca 4500
   tgctggggtt aatagaatat gtcagtttat cacttgtcgc ttatttagct ttaaaataaa 4560
   aattaatagg caaagcaatg gaatatttgc agtttcacct aaagagcagc ataaggaggc 4620
   gggaatccaa agtgaagttg tttgatatgg tctacttctt ttttggaatt tcctgaccat 4680
   taattaaaga attggatttg caagtttgaa aactggaaaa gcaagagatg ggatgccata 4740
   atagtaaaca gcccttgtgt tggatgtaac ccaatcccag atttgagtgt gtgttgatta 4800
   tttttttgtc ttccactttt ctattatgtg taaatcactt ttatttctgc agacattttc 4860
   ctctcagata ggatgacatt ttgttttgta ttattttgtc tttcctcatg aatgcactga 4920
   taatatttta aatgctctat tttaagatct cttgaatctg tttttttttt ttttaatttg 4980
   ggggttctgt aaggtcttta tttcccataa gtaaatattg ccatgggagg ggggtggagg 5040
   tggcaaggaa ggggtgaagt gctagtatgc aagtgggcag caattatttt tgtgttaatc 5100
   agcagtacaa tttgatcgtt ggcatggtta aaaaatggaa tataagatta gctgttttgt 5160
   attttgatga ccaattacgc tgtattttaa cacgatgtat gtctgttttt gtggtgctct 5220
   agtggtaaat aaattatttc gatgatatgt ggatgtcttt ttcctatcag taccatcatc 5280
   gagtctagaa aacacctgtg atgcaataag actatctcaa gctggaaaag tcataccacc 5340
   tttccgattg ccctctgtgc tttctccctt aaggacagtc acttcagaag tcatgcttta 5400
   aagcacaaga gtcaggccat atccatcaag gatagaagaa atccctgtgc cgtcttttta 5460
   ttcccttatt tattgctatt tggtaattgt ttgagattta gtttccatcc agcttgactg 5520
   ccgaccagaa aaaatgcaga gagatgtttg caccatgctt tggctttctg gttctatgtt 5580
   ctgccaacgc cagggccaaa agaactggtc tagacagtat cccctgtagc cccataactt 5640
   ggatagttgc tgagccagcc agatataaca agagccacgt gctttctggg gttggttgtt 5700
   tgggatcagc tacttgcctg tcagtttcac tggtaccact gcaccacaaa caaaaaaacc 5760
   caccctattt cctccaattt ttttggctgc tacctacaag accagactcc tcaaacgagt 5820
   tgccaatctc ttaataaata ggattaataa aaaaagtaat tgtgactcaa aaaaaaaaaa 5880
   aa 5882
<210> 2
   <211> 37
   <212> DNA
   <213> Artificial
<220>
   <223> primer
<400> 2
   tcgaggacct gacacgaggg tggaaatgga tacacga 37
<210> 3
   <211> 20
   <212> DNA
   <213> Artificial
<220>
   <223> primer
<400> 3
   ggagaagcag atgcttctgg 20
<210> 4
   <211> 22
   <212> DNA
   <213> Artificial
<220>
   <223> primer
<400> 4
   ccaaacaact ctccttgatc ta 22
<210> 5
   <211> 20
   <212> DNA
   <213> Artificial
<220>
   <223> primer
<400> 5
   cccttatggt ttcttgggat 20
<210> 6
   <211> 20
   <212> DNA
   <213> Artificial
<220>
   <223> primer
<400> 6
   gtgaccatgc aattgagatg 20
<210> 7
   <211> 20
   <212> DNA
   <213> Artificial
<220>
   <223> primer
<400> 7
   gaatggcttc accataaagg 20
<210> 8
   <211> 20
   <212> DNA
   <213> Artificial
<220>
   <223> primer
<400> 8
   cagctgatgc tgctttggtg 20
<210> 9
   <211> 22
   <212> DNA
   <213> Artificial
<220>
   <223> primer
<400> 9
   cacttgagat tacaataaag cc 22
<210> 10
   <211> 19
   <212> DNA
   <213> Artificial
<220>
   <223> primer
<400> 10
   gcctactcag tgctgtgac 19
<210> 11
   <211> 20
   <212> DNA
   <213> Artificial
<220>
   <223> primer
<400> 11
   aagggcgatc ccgggagctg 20
<210> 12
   <211> 20
   <212> DNA
   <213> Artificial
<220>
   <223> primer
<400> 12
   gttcaaggtt gcagtgagct 20
<210> 13
   <211> 18
   <212> DNA
   <213> Artificial
<220>
   <223> primer
<400> 13
   caagttttgg ttacctgc 18
<210> 14
   <211> 22
   <212> DNA
   <213> Artificial
<220>
   <223> primer
<400> 14
   caaccatatc actgtccaaa tg 22
<210> 15
   <211> 18
   <212> DNA
   <213> Artificial
<220>
   <223> primer
<400> 15
   tgcactctat ctggcctt 18
<210> 16
   <211> 18
   <212> DNA
   <213> Artificial
<220>
   <223> primer
<400> 16
   ccagtaccca tacatgtg 18
<210> 17
   <211> 19
   <212> DNA
   <213> Artificial
<220>
   <223> primer
<400> 17
   actgatgaac caaggctct 19

## Description

### FIELD OF THE INVENTION:

The present invention relates to methods for the diagnosis and the treatment of familial thoracic aortic aneurysms caused by *TGFB2* loss of function mutations.

### BACKGROUND OF THE INVENTION:

Thoracic aortic aneurysms can lead to acute aortic dissections or ruptures, and death due to these complications is common¹. A number of genetic syndromes predispose to thoracic aortic disease, including Marfan syndrome (MFS, MIM 154700), Loeys-Dietz syndrome (LDS, MIM 609192, 608967, 610168, 610380) and Aneurysms-Osteoarthritis Syndrome (AOS, MIM 613795)²⁻⁴. Systemic complications are shared among these syndromes, including skeletal, craniofacial and skin manifestations. LDS is caused by mutations in the genes encoding the TGF-β receptors type I and II (*TGFBR1* and *TGFBR2,* respectively), which bind TGF-β and initiate cellular signaling. AOS results from mutations in *SMAD3* (SMAD family members 3), which encodes a protein critical for cellular signaling downstream of the TGF-β receptors after ligand binding. Despite the fact that the majority of mutations in these genes are predicted to disrupt proper function of these signaling proteins, diseased aortic tissue from these patients show increased nuclear phosphorylated SMAD2 (pSMAD2) in aortic smooth muscle cells (SMCs), suggesting increased TGF-β cellular signaling^{3,4}. Although MFS is caused by mutations in *FBN1*, encoding an extracellular matrix protein called fibillin-1, excessive TGF-β signaling is also evident in aortic tissue from MFS patients and mouse models^{5,6}.

### SUMMARY OF THE INVENTION:

The present invention relates to methods for the diagnosis and the treatment of familial thoracic aortic aneurysms caused by TGFB2 loss of function mutations.

### DETAILED DESCRIPTION OF THE INVENTION:

A predisposition for thoracic aortic aneurysms leading to acute aortic dissections can be inherited in families in an autosomal dominant manner. Genome-wide linkage analysis of two large unrelated families with thoracic aortic disease, followed by whole exome sequencing of affected relatives, identified causative mutations in *TGFB2.* These mutations, a frameshift mutation in exon 6 and a nonsense mutation in exon 4, segregated with disease with a combined LOD score of 7.7. Sanger sequencing of 276 probands from families with inherited thoracic aortic disease identified two additional *TGFB2* mutations. *TGFB2* encodes the transforming growth factor beta-2 (TGF-β2) and the mutations are predicted to cause haploinsufficiency for *TGFB2*, but aortic tissue from cases paradoxically shows increased TGF-β2 expression and immunostaining. Thus, haploinsufficiency of *TGFB2* predisposes to thoracic aortic disease, suggesting the initial pathway driving disease is decreased cellular TGF-β2 levels leading to a secondary increase in TGF-β2 production in the diseased aorta.

Accordingly the present invention relates to a method for determining whether a subject is predisposed to thoracic aortic aneurysms comprising detecting a *TGFB2* loss of function mutation wherein the presence of the mutation indicated that the subject is predisposed to thoracic aortic aneurysms.

The terms "subject," and "patient," used interchangeably herein, refer to a male or female. Typically the subject can be one who exhibits one or more risk factors for thoracic aortic aneurysm, or a subject who does not exhibit thoracic aortic aneurysm risk factor. Typically, the subject may be one that is at risk of developing thoracic aortic aneurysm, as defined by clinical indicia such as for example age, a family history of thoracic aortic aneurysm.

As used herein the term "*TGFB2*" has its general meaning in the art and refers to the transforming growth factor beta-2 (TGF-β2). Genomic sequence is accessible to Genbakn via NG_027721.1 access number. mRNA and protein sequences are accessible via NM_001135599.2 (isoform 1) and NM_003238.3 (isoform 2) (SEQ ID NO:1) NP_001129071.1 (isoform 1) and NP_003229.1 (isoform 2). Typically, a nucleotide sequence for TGFB2 is SEQ ID NO:1.
SEQ ID NO:1

As used herein the term "*TGFB2* loss of function mutation" refers to any mutation that leads to a haploinsufficiency for *TGFB2.* Typically, the mutation leads to a premature stop codon and accordingly may be a "non sense" mutation (i.e. replaces an amino acid codon with a stop codon) or the mutation may lead to a frameshift (deletion or addition) in the translation of the transcript resulting in a premature stop codon. In a particular embodiment, the *TGFB2* loss of function mutation is located in exon 4, 5, 6 or 7. In another particular embodiment, the *TGFB2* loss of function mutation is the 5 base pair deletion c.1021_1025delTACAA in exon 6 of the gene, the nonsense p.Cys229* mutation, the nonsense p.Glu102* mutation or frameshift duplication (c.873_888dup) leading to p.Asn297*.

Generally the mutation is identified in the subject by comparing the sequence of a nucleic acid or polypeptide expressed by said subject with the corresponding nucleic acid or polypeptide expressed in a control population.

According to a first embodiment, said mutation may be detected by analyzing a *TGFB2* nucleic acid molecule. In the context of the invention, *TGFB2* nucleic acid molecules include mRNA, genomic DNA and cDNA derived from mRNA. DNA or RNA can be single stranded or double stranded. These may be utilized for detection by amplification and/or hybridization with a probe, for instance.

The nucleic acid sample may be obtained from any cell source or tissue biopsy. Nonlimiting examples of cell sources available include without limitation blood cells, buccal cells, epithelial cells, fibroblasts, or any cells present in a tissue obtained by biopsy. Cells may also be obtained from body fluids, such as blood, plasma, serum, lymph, etc. DNA may be extracted using any methods known in the art, such as described in Sambrook et al., 1989. RNA may also be isolated, for instance from tissue biopsy, using standard methods well known to the one skilled in the art such as guanidium thiocyanate-phenol-chloroform extraction.

Typically *TGFB2* loss of function mutations according to the invention may be detected in a RNA or DNA sample, preferably after amplification. For instance, the isolated RNA may be subjected to coupled reverse transcription and amplification, such as reverse transcription and amplification by polymerase chain reaction (RT-PCR), using specific oligonucleotide primers that are specific for a mutated site or that enable amplification of a region containing the mutated site. According to a first alternative, conditions for primer annealing may be chosen to ensure specific reverse transcription (where appropriate) and amplification; so that the appearance of an amplification product be a diagnostic of the presence of a particular *TGFB2* mutation. Otherwise, RNA may be reverse-transcribed and amplified, or DNA may be amplified, after which a mutated site may be detected in the amplified sequence by hybridization with a suitable probe or by direct sequencing, or any other appropriate method known in the art. For instance, a cDNA obtained from RNA may be cloned and sequenced to identify a mutation in TGFB2 sequence.

Actually numerous strategies for genotype analysis are available. Briefly, the nucleic acid molecule may be tested for the presence or absence of a restriction site. When a base substitution mutation creates or abolishes the recognition site of a restriction enzyme, this allows a simple direct PCR test for the mutation. Further strategies include, but are not limited to, direct sequencing, restriction fragment length polymorphism (RFLP) analysis; hybridization with allele-specific oligonucleotides (ASO) that are short synthetic probes which hybridize only to a perfectly matched sequence under suitably stringent hybridization conditions; allele-specific PCR; PCR using mutagenic primers; ligase-PCR, HOT cleavage; denaturing gradient gel electrophoresis (DGGE), temperature denaturing gradient gel electrophoresis (TGGE), single-stranded conformational polymorphism (SSCP) and denaturing high performance liquid chromatography. Direct sequencing may be accomplished by any method, including without limitation chemical sequencing, using the Maxam-Gilbert method ; by enzymatic sequencing, using the Sanger method ; mass spectrometry sequencing ; sequencing using a chip-based technology; and real-time quantitative PCR. Preferably, DNA from a subject is first subjected to amplification by polymerase chain reaction (PCR) using specific amplification primers. However several other methods are available, allowing DNA to be studied independently of PCR, such as the rolling circle amplification (RCA), the InvaderTMassay, or oligonucleotide ligation assay (OLA). OLA may be used for revealing base substitution mutations. According to this method, two oligonucleotides are constructed that hybridize to adjacent sequences in the target nucleic acid, with the join sited at the position of the mutation. DNA ligase will covalently join the two oligonucleotides only if they are perfectly hybridized.

Therefore, useful nucleic acid molecules, in particular oligonucleotide probes or primers, according to the present invention include those which specifically hybridize the regions where the mutations are located.

Oligonucleotide probes or primers may contain at least 10, 15, 20 or 30 nucleotides. Their length may be shorter than 400, 300, 200 or 100 nucleotides.

According to another aspect of the invention, the *TGFB2* loss of function mutation is detected by contacting the DNA of the subject with a nucleic acid probe, which is optionally labeled.

Primers may also be useful to amplify or sequence the portion of the *TGFB2* gene containing the mutated positions of interest.

Such probes or primers are nucleic acids that are capable of specifically hybridizing with a portion of the *TGFB2* gene sequence containing the mutated positions of interest. That means that they are sequences that hybridize with the portion mutated *TGFB2* nucleic acid sequence to which they relate under conditions of high stringency.

According to a second embodiment the *TGFB2* loss of function mutation may be detected at the protein level.

Said mutation may be detected according to any appropriate method known in the art. In particular a sample, such as a tissue biopsy, obtained from a subject may be contacted with antibodies specific of the mutated form of TGFB2, i.e. antibodies that are capable of distinguishing between a mutated form of TGFB2 and the wild-type protein (or any other protein), to determine the presence or absence of a TGFB2 specified by the antibody.

Antibodies that specifically recognize a mutated TGFB2 also make part of the invention. The antibodies are specific of mutated TGFB2, that is to say they do not cross-react with the wild-type TGFB2.

The antibodies of the present invention may be monoclonal or polyclonal antibodies, single chain or double chain, chimeric antibodies, humanized antibodies, or portions of an immunoglobulin molecule, including those portions known in the art as antigen binding fragments Fab, Fab', F(ab')2 and F(v). They can also be immunoconjugated, e.g. with a toxin, or labelled antibodies.

Whereas polyclonal antibodies may be used, monoclonal antibodies are preferred for they are more reproducible in the long run.

Procedures for raising "polyclonal antibodies" are also well known. Polyclonal antibodies can be obtained from serum of an animal immunized against the appropriate antigen, which may be produced by genetic engineering for example according to standard methods well-known by one skilled in the art. Typically, such antibodies can be raised by administering mutated TGFB2 subcutaneously to New Zealand white rabbits which have first been bled to obtain pre-immune serum. The antigens can be injected at a total volume of 100 µl per site at six different sites. Each injected material may contain adjuvants with or without pulverized acrylamide gel containing the protein or polypeptide after SDS-polyacrylamide gel electrophoresis. The rabbits are then bled two weeks after the first injection and periodically boosted with the same antigen three times every six weeks. A sample of serum is then collected 10 days after each boost. Polyclonal antibodies are then recovered from the serum by affinity chromatography using the corresponding antigen to capture the antibody. This and other procedures for raising polyclonal antibodies are disclosed by Harlow et al. (1988).

A "monoclonal antibody" in its various grammatical forms refers to a population of antibody molecules that contains only one species of antibody combining site capable of immunoreacting with a particular epitope. A monoclonal antibody thus typically displays a single binding affinity for any epitope with which it immunoreacts. A monoclonal antibody may therefore contain an antibody molecule having a plurality of antibody combining sites, each immunospecific for a different epitope, e.g. a bispecific monoclonal antibody. Although historically a monoclonal antibody was produced by immortalization of a clonally pure immunoglobulin secreting cell line, a monoclonally pure population of antibody molecules can also be prepared by the methods of the present invention.

Laboratory methods for preparing monoclonal antibodies are well known in the art (see, for example, Harlow et al., 1988). Monoclonal antibodies (mAbs) may be prepared by immunizing purified mutated TGFB2 into a mammal, e.g. a mouse, rat, human and the like mammals. The antibody-producing cells in the immunized mammal are isolated and fused with myeloma or heteromyeloma cells to produce hybrid cells (hybridoma). The hybridoma cells producing the monoclonal antibodies are utilized as a source of the desired monoclonal antibody. This standard method of hybridoma culture is described in Kohler and Milstein (1975).

While mAbs can be produced by hybridoma culture the invention is not to be so limited. Also contemplated is the use of mAbs produced by an expressing nucleic acid cloned from a hybridoma of this invention. That is, the nucleic acid expressing the molecules secreted by a hybridoma of this invention can be transferred into another cell line to produce a transformant. The transformant is genotypically distinct from the original hybridoma but is also capable of producing antibody molecules of this invention, including immunologically active fragments of whole antibody molecules, corresponding to those secreted by the hybridoma. See, for example, U.S. Pat. No. 4,642,334 to Reading; PCT Publication No.; European Patent Publications No. 0239400 to Winter et al. and No. 0125023 to Cabilly et al.

Antibody generation techniques not involving immunisation are also contemplated such as for example using phage display technology to examine naive libraries (from non-immunised animals); see Barbas et al. (1992), and Waterhouse et al. (1993).

Antibodies raised against mutated TGFB2 may be cross reactive with wild-type TGFB2. Accordingly a selection of antibodies specific for mutated TGFB2 is required. This may be achieved by depleting the pool of antibodies from those that are reactive with the wild-type TGFB2, for instance by submitting the raised antibodies to an affinity chromatography against wild-type TGFB2.

Alternatively, binding agents other than antibodies may be used for the purpose of the invention. These may be for instance aptamers, which are a class of molecule that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by Exponential enrichment (SELEX) of a random sequence library, as described in Tuerk C. and Gold L., 1990. The random sequence library is obtainable by combinatorial chemical synthesis of DNA. In this library, each member is a linear oligomer, eventually chemically modified, of a unique sequence. Possible modifications, uses and advantages of this class of molecules have been reviewed in Jayasena S.D., 1999. Peptide aptamers consists of a conformationally constrained antibody variable region displayed by a platform protein, such as E. coli Thioredoxin A that are selected from combinatorial libraries by two hybrid methods (Colas et al., 1996).

Probe, primers, aptamers or antibodies of the invention may be labelled with a detectable molecule or substance, such as a fluorescent molecule, a radioactive molecule or any others labels known in the art. Labels are known in the art that generally provide (either directly or indirectly) a signal.

The term "labelled", with regard to the probe, primers, aptamers or antibodies of the invention, is intended to encompass direct labelling of the the probe, primers, aptamers or antibodies of the invention by coupling (i.e., physically linking) a detectable substance to the the probe, primers, aptamers or antibodies of the invention, as well as indirect labeling of the probe, primers, aptamers or antibodies of the invention by reactivity with another reagent that is directly labeled. Other examples of detectable substances include but are not limited to radioactive agents or a fluorophore (e.g. fluorescein isothiocyanate (FITC) or phycoerythrin (PE) or Indocyanine (Cy5)). Examples of indirect labeling include detection of a primary antibody using a fluorescently labeled secondary antibody and end-labeling of a DNA probe with biotin such that it can be detected with fluorescently labeled streptavidin. An antibody or aptamer of the invention may be labelled with a radioactive molecule by any method known in the art. For example radioactive molecules include but are not limited radioactive atom for scintigraphic studies such as I123, I124, In111, Re186, Re188.

The present invention further provides kits suitable for determining at least one *TGFB2* loss of function mutation according to the invention.

The kits may include the following components:
(i) a probe, usually made of DNA, and that may be pre-labelled. Alternatively, the probe may be unlabelled and the ingredients for labelling may be included in the kit in separate containers ; and
(ii) hybridization reagents : the kit may also contain other suitably packaged reagents and materials needed for the particular hybridization protocol, including solid-phase matrices, if applicable, and standards.

In another embodiment, the kits may include :
(i) sequence determination or amplification primers : sequencing primers may be pre-labelled or may contain an affinity purification or attachment moiety ; and
(ii) sequence determination or amplification reagents : the kit may also contain other suitably packaged reagents and materials needed for the particular sequencing amplification protocol. In one preferred embodiment, the kit comprises a panel of sequencing or amplification primers, whose sequences correspond to sequences adjacent to at least one of the polymorphic positions, as well as a means for detecting the presence of each polymorphic sequence.

Alternatively, the kit of the invention may comprise a labelled compound or agent capable of detecting the mutated polypeptide of the invention (e.g., an antibody or aptamers as described above which binds the polypeptide). For example, the kit may comprise (1) a first antibody (e.g., attached to a solid support) which binds to a polypeptide comprising a mutation of the invention; and, optionally, (2) a second, different antibody which binds to either the polypeptide or the first antibody and is conjugated to a detectable agent.

The kit can also comprise, e.g., a buffering agent, a preservative, or a protein stabilizing agent. The kit can also comprise components necessary for detecting the detectable agent (e.g., an enzyme or a substrate). The kit can also contain a control sample or a series of control samples which can be assayed and compared to the test sample contained. Each component of the kit is usually enclosed within an individual container and all of the various containers are within a single package along with instructions for observing whether the tested subject is suffering from or is at risk of developing a bone mineral density related disease.

A further object to the invention relates to a transforming growth factor beta-2 (TGF-β2) polypeptide for use in the prophylactic treatment of a subject who has been considered as predisposed to thoracic aortic aneurysms by the method of the invention (i.e a subject having one TGB2 loss of function mutation according to the invention).

The terms "prophylaxis" or "prophylactic use" and "prophylactic treatment" as used herein, refer to any medical or public health procedure whose purpose is to prevent a disease. As used herein, the terms "prevent", "prevention" and "preventing" refer to the reduction in the risk of acquiring or developing a given condition, or the reduction or inhibition of the recurrence or said condition in a subject who is not ill, but who has been or may be near a subject with the disease.

The term " TGF-β2 polypeptide" has its general meaning in the art and includes naturally occurring TGF-β2 (i.e TGF-β2 with no loss of function mutation) and function conservative variants and modified forms thereof. The TGF-β2 can be from any source, but typically is a mammalian (e.g., human and non-human primate) TGF-B2, and more particularly a human TGF-β2. "Function-conservative variants" are those in which a given amino acid residue in a polypeptide has been changed without altering the overall conformation and function of the polypeptide, including, but not limited to, replacement of an amino acid with one having similar properties (such as, for example, polarity, hydrogen bonding potential, acidic, basic, hydrophobic, aromatic, and the like). Amino acids other than those indicated as conserved may differ in a protein so that the percent protein or amino acid sequence similarity between any two proteins of similar function may vary and may be, for example, from 70 % to 99 % as determined according to an alignment scheme such as by the Cluster Method, wherein similarity is based on the MEGALIGN algorithm. A "function-conservative variant" also includes a polypeptide which has at least 60 % amino acid identity as determined by BLAST or FASTA algorithms, preferably at least 75 %, most preferably at least 85%, and even more preferably at least 90 %, and which has the same or substantially similar properties or functions as the native or parent protein to which it is compared.

In specific embodiments, it is contemplated that TGF-β2 polypeptides used in the therapeutic methods of the present invention may be modified in order to improve their therapeutic efficacy. Such modification of therapeutic compounds may be used to decrease toxicity, increase circulatory time, or modify biodistribution. For example, the toxicity of potentially important therapeutic compounds can be decreased significantly by combination with a variety of drug carrier vehicles that modify biodistribution.

A strategy for improving drug viability is the utilization of water-soluble polymers. Various water-soluble polymers have been shown to modify biodistribution, improve the mode of cellular uptake, change the permeability through physiological barriers; and modify the rate of clearance from the body. To achieve either a targeting or sustained-release effect, water-soluble polymers have been synthesized that contain drug moieties as terminal groups, as part of the backbone, or as pendent groups on the polymer chain.

Polyethylene glycol (PEG) has been widely used as a drug carrier, given its high degree of biocompatibility and ease of modification. Attachment to various drugs, proteins, and liposomes has been shown to improve residence time and decrease toxicity. PEG can be coupled to active agents through the hydroxyl groups at the ends of the chain and via other chemical methods; however, PEG itself is limited to at most two active agents per molecule. In a different approach, copolymers of PEG and amino acids were explored as novel biomaterials which would retain the biocompatibility properties of PEG, but which would have the added advantage of numerous attachment points per molecule (providing greater drug loading), and which could be synthetically designed to suit a variety of applications.

Those of skill in the art are aware of PEGylation techniques for the effective modification of drugs. For example, drug delivery polymers that consist of alternating polymers of PEG and tri-functional monomers such as lysine have been used by VectraMed (Plainsboro, N.J.). The PEG chains (typically 2000 daltons or less) are linked to the a- and e-amino groups of lysine through stable urethane linkages. Such copolymers retain the desirable properties of PEG, while providing reactive pendent groups (the carboxylic acid groups of lysine) at strictly controlled and predetermined intervals along the polymer chain. The reactive pendent groups can be used for derivatization, cross-linking, or conjugation with other molecules. These polymers are useful in producing stable, long-circulating pro-drugs by varying the molecular weight of the polymer, the molecular weight of the PEG segments, and the cleavable linkage between the drug and the polymer. The molecular weight of the PEG segments affects the spacing of the drug/linking group complex and the amount of drug per molecular weight of conjugate (smaller PEG segments provides greater drug loading). In general, increasing the overall molecular weight of the block co-polymer conjugate will increase the circulatory half-life of the conjugate. Nevertheless, the conjugate must either be readily degradable or have a molecular weight below the threshold-limiting glomular filtration (e.g., less than 45 kDa).

In addition, to the polymer backbone being important in maintaining circulatory half-life, and biodistribution, linkers may be used to maintain the therapeutic agent in a pro-drug form until released from the backbone polymer by a specific trigger, typically enzyme activity in the targeted tissue. For example, this type of tissue activated drug delivery is particularly useful where delivery to a specific site of biodistribution is required and the therapeutic agent is released at or near the site of pathology. Linking group libraries for use in activated drug delivery are known to those of skill in the art and may be based on enzyme kinetics, prevalence of active enzyme, and cleavage specificity of the selected disease-specific enzymes (see e.g., technologies of established by VectraMed, Plainsboro, N.J.). Such linkers may be used in modifying the TGF-β2 -derived proteins described herein for therapeutic delivery.

In another particular embodiment the TGF-β2 polypeptide is fused a Fc domain of an immunoglobulin. Suitable immunoglobins are IgG, IgM, IgA, IgD, and IgE. IgG and IgA are preferred IgGs are most preferred, e.g. an IgG1. Said Fc domain may be a complete Fc domain or a function-conservative variant thereof. The TGF-B2 polypeptide of the invention may be linked to the Fc domain by a linker. The linker may consist of about 1 to 100, preferably 1 to 10 amino acid residues.

According to the invention, TGF-β2 polypeptide may be produced by conventional automated peptide synthesis methods or by recombinant expression. General principles for designing and making proteins are well known to those of skill in the art.

TGF-β2 polypeptides of the invention may be synthesized in solution or on a solid support in accordance with conventional techniques. Various automatic synthesizers are commercially available. TGF-β2 polypeptides of the invention may also be synthesized by solid-phase technology employing an exemplary peptide synthesizer. The purity of any given protein; generated through automated peptide synthesis or through recombinant methods may be determined using reverse phase HPLC analysis. Chemical authenticity of each peptide may be established by any method well known to those of skill in the art.

As an alternative to automated peptide synthesis, recombinant DNA technology may be employed wherein a nucleotide sequence which encodes a protein of choice is inserted into an expression vector, transformed or transfected into an appropriate host cell and cultivated under conditions suitable for expression as described herein below. Recombinant methods are especially preferred for producing longer polypeptides.

A variety of expression vector/host systems may be utilized to contain and express the peptide or protein coding sequence. These include but are not limited to microorganisms such as bacteria transformed with recombinant bacteriophage, plasmid or cosmid DNA expression vectors; yeast transformed with yeast expression vectors; insect cell systems infected with virus expression vectors (e.g., baculovirus); plant cell systems transfected with virus expression vectors (e.g., cauliflower mosaic virus, CaMV; tobacco mosaic virus, TMV) or transformed with bacterial expression vectors (e.g., Ti or pBR322 plasmid); or animal cell systems. Those of skill in the art are aware of various techniques for optimizing mammalian expression of proteins. Mammalian cells that are useful in recombinant protein productions include but are not limited to VERO cells, HeLa cells, Chinese hamster ovary (CHO) cell lines, COS cells (such as COS-7), W138, BHK, HepG2, 3T3, RIN, MDCK, A549, PC12, K562 and 293 cells. Exemplary protocols for the recombinant expression of the peptide substrates or fusion polypeptides in bacteria, yeast and other invertebrates are known to those of skill in the art and a briefly described herein below. Mammalian host systems for the expression of recombinant proteins also are well known to those of skill in the art. Host cell strains may be chosen for a particular ability to process the expressed protein or produce certain post-translation modifications that will be useful in providing protein activity. Such modifications of the polypeptide include, but are not limited to, acetylation, carboxylation, glycosylation, phosphorylation, lipidation and acylation. Post-translational processing which cleaves a "prepro" form of the protein may also be important for correct insertion, folding and/or function. Different host cells such as CHO, HeLa, MDCK, 293, WI38, and the like have specific cellular machinery and characteristic mechanisms for such post-translational activities and may be chosen to ensure the correct modification and processing of the introduced, foreign protein.

In the recombinant production of the TGF-β2 -derived proteins of the invention, it would be necessary to employ vectors comprising polynucleotide molecules for encoding the TGF-β2 -derived proteins. Methods of preparing such vectors as well as producing host cells transformed with such vectors are well known to those skilled in the art. The polynucleotide molecules used in such an endeavor may be joined to a vector, which generally includes a selectable marker and an origin of replication, for propagation in a host. These elements of the expression constructs are well known to those of skill in the art. Generally, the expression vectors include DNA encoding the given protein being operably linked to suitable transcriptional or translational regulatory sequences, such as those derived from a mammalian, microbial, viral, or insect genes. Examples of regulatory sequences include transcriptional promoters, operators, or enhancers, mRNA ribosomal binding sites, and appropriate sequences which control transcription and translation.

The terms "expression vector," "expression construct" or "expression cassette" are used interchangeably throughout this specification and are meant to include any type of genetic construct containing a nucleic acid coding for a gene product in which part or all of the nucleic acid encoding sequence is capable of being transcribed.

The choice of a suitable expression vector for expression of the peptides or polypeptides of the invention will of course depend upon the specific host cell to be used, and is within the skill of the ordinary artisan.

Expression requires that appropriate signals be provided in the vectors, such as enhancers/promoters from both viral and mammalian sources that may be used to drive expression of the nucleic acids of interest in host cells. Usually, the nucleic acid being expressed is under transcriptional control of a promoter. A "promoter" refers to a DNA sequence recognized by the synthetic machinery of the cell, or introduced synthetic machinery, required to initiate the specific transcription of a gene. Nucleotide sequences are operably linked when the regulatory sequence functionally relates to the DNA encoding the protein of interest (i.e., TGF-β2, a variant and the like). Thus, a promoter nucleotide sequence is operably linked to a given DNA sequence if the promoter nucleotide sequence directs the transcription of the sequence.

Another aspect of the invention relates to a nucleic acid molecule encoding for a TGF-β2 polypeptide for use in the prophylactic treatment of a subject who has been considered as predisposed to thoracic aortic aneurysms by the method of the invention (i.e a subject having one *TGB2* loss of function mutation according to the invention).

Typically, said nucleic acid is a DNA or RNA molecule, which may be included in any suitable vector, such as a plasmid, cosmid, episome, artificial chromosome, phage or a viral vector as above described. So, a further object of the invention relates to a vector comprising a nucleic acid encoding for an TGF-β2polypeptide for use in the prophylactic treatment of a subject who has been considered as predisposed to thoracic aortic aneurysms by the method of the invention (i.e a subject having one *TGB2* loss of function mutation according to the invention).

The present invention relates to a method for preventing a thoracic aortic aneurysm in a subject considered as predisposed to thoracic aortic aneurysms by the method of the invention (i.e a subject having one *TGB2* loss of function mutation according to the invention) comprising the step of administrating said patient with therapeutically effective amount of a TGF-β2 polypeptide (or nucleic acid encoding for TGF-β2 polypeptides).

By a "therapeutically effective amount" is meant a sufficient amount of TGF-β2 polypeptides (or nucleic acid encoding for a TGF-β2 polypeptide) to prevent a bacterial superinfection post-influenza at a reasonable benefit/risk ratio applicable to any medical treatment.

It will be understood that the total daily usage of the compounds and compositions of the present invention will be decided by the attending physician within the scope of sound medical judgment. The specific therapeutically effective dose level for any particular patient will depend upon a variety of factors including the age, body weight, general health, sex and diet of the patient; the time of administration, route of administration, and rate of excretion of the specific compound employed; the duration of the treatment; drugs used in combination or coincidental with the specific polypeptide employed; and like factors well known in the medical arts. For example, it is well known within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the patient to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

The TGF-β2 polypeptides of the invention (or the nucleic acid encoding thereof) may be combined with pharmaceutically acceptable excipients, and optionally sustained-release matrices, such as biodegradable polymers, to form phamaceutical compositions.

"Pharmaceutically" or "pharmaceutically acceptable" refer to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

In the pharmaceutical compositions of the present invention for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The TGF-B2 polypeptide (or nucleic acid encoding thereof) can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active polypeptides in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion. Some variation in dosage will necessarily occur depending on the condition of the subject being treated. The person responsible for administration will, in any event, determine the appropriate dose for the individual subject.

The TGF-β2 polypeptide (or nucleic acid encoding thereof) may be formulated within a therapeutic mixture to comprise about 0.0001 to 1.0 milligrams, or about 0.001 to 0.1 milligrams, or about 0.1 to 1.0 or even about 10 milligrams per dose or so. Multiple doses can also be administered.

The invention will be further illustrated by the following figures and examples. However, these examples and figures should not be interpreted in any way as limiting the scope of the present invention.

### FIGURES:

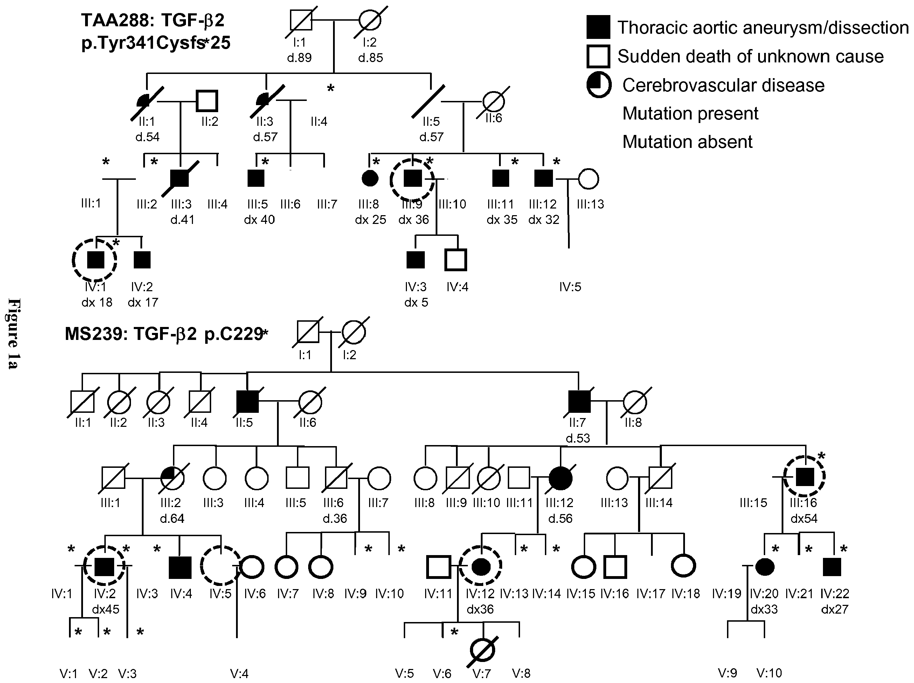

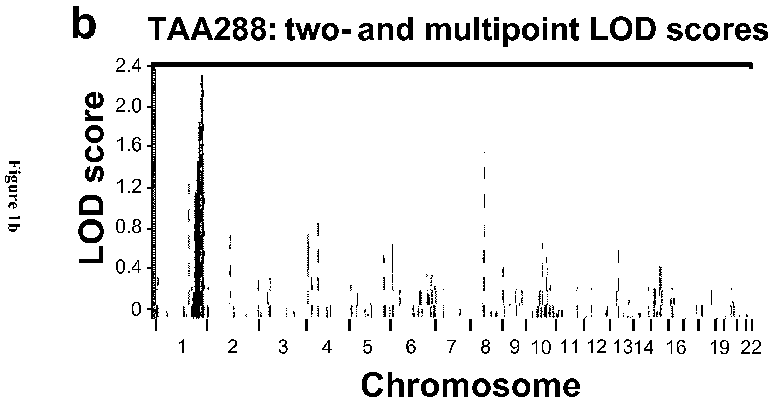

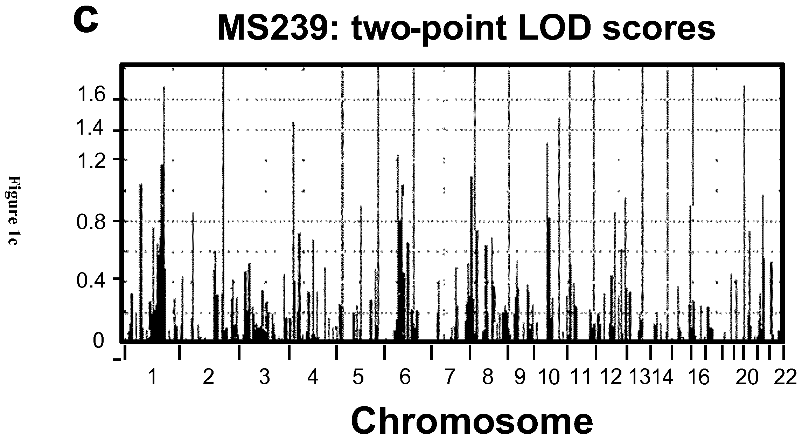

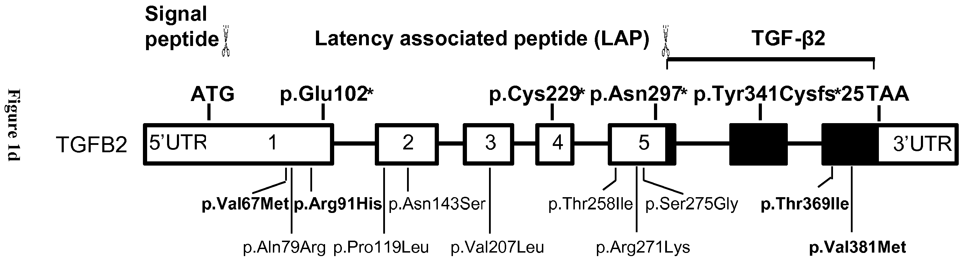

**Figure 1****.** Identification of *TGFB2* as the causative gene responsible for thoracic aortic disease in families TAA288 and MS239. (a) Pedigrees of family TAA288 and MS239 with the legend indicating the disease and mutation status of the family members. The age at diagnosis of aortic root enlargement and/or dissection ("dx") is shown in years and "d" indicates age at death. A single asterisk indicates individuals whose DNA was used in genome-wide mapping. DNA from the circled individuals was used for exome sequencing. (b) Parametric two-point and multi-point LOD score profile for thoracic aortic aneurysms and dissections (TAAD) across the human genome in family TAA288 based on the Affymetrix 50GeneChips Hind array data. The parametric two-point and multi-point LOD scores are on the y-axis and are correlated to physical location of human chromosome on the x-axis. (c) Parametric two-point LOD score profile for TAAD across the human genome in family MS239. (d) Schematic representation of the *TGFB2* gene and the protein domains and preproprotein proteolytic processing sites for mature TGF-β2. Boxes represent exons 1-7 with the untranslated regions (UTRs) and the open reading frame designated. The proteolytic sites of TGF-β2 preproprotein are marked with scissors symbol. Proteolytic cleavage sites remove the signal peptides from the amino-terminus and release the mature TGF-β2 from the latent associated peptide. The *TGFB2* mutations identified in this study are indicated in pink type. Below the gene diagram are the rare variants found in the NHLBI exome sequencing variant server (http://evs.gs.washington.edu/EVS/); blue type designates variants predicted to be possibly or probably damaging by PolyPhen-2 analysis and black type designates variants predicted to be benign.

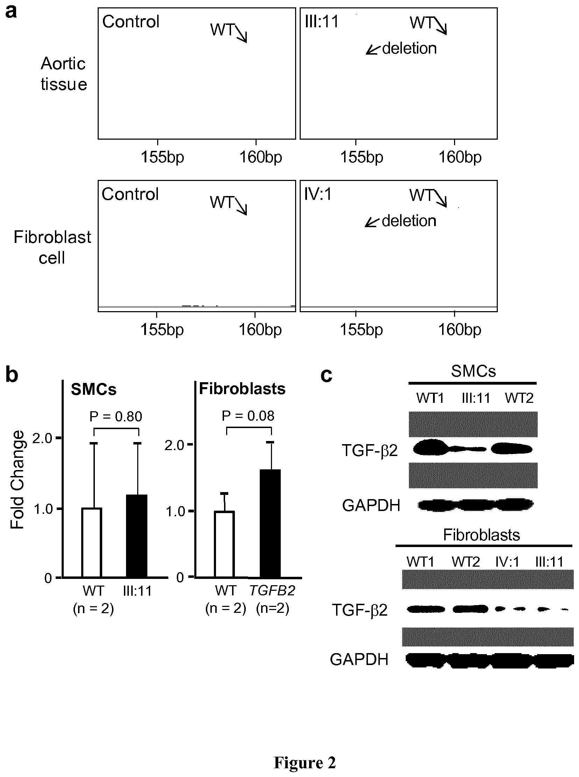

**Figure 2****.** Transcript and protein analysis of the *TGFB2* mutation p.Tyr341Cysfs*25 in exon 6. (a) RT-PCR of RNA extracted from normal ascending aortic specimens (n=2) or fibroblast cell cultures (n=2) showed only one PCR product at 160 bp. RT-PCR of RNA extracted from ascending aortic specimen of a patient (III:11) or fibroblast cell culture (IV: 1) from family TAA288 demonstrated that the transcript from both normal and 5 bp deleted allele were present. (b) Expression of *TGFB2* was quantified in aortic SMCs and dermal fibroblasts explanted from *TGFB2* mutation carriers and matched controls using quantitative-PCR. *TGFB2* expression levels between patients and controls were similar in both SMCs and fibroblasts. *TGFB2* levels are standardized to *GAPDH* messages. The relative expression values were determined via the ΔΔCt method, and assays were performed in triplicate. Data are expressed as mean ± standard error of the mean for pooled experimental results. (c) Immunoblot analysis of TGF-β2 proprotein in cellular lysates of aortic SMCs and fibroblasts from patients (III:11 and IV:1, TAA288 family) and two normal controls. A protein band at 47kD, the molecular weight of the proprotein of TGF-β2, was identified with immunoblot analysis in both the patient and control lysates using a polyclonal antibody specific for TGF-β2. Decreased intensity of this band was found in patients' cells, both SMCs and fibroblasts, when compared with control cells.

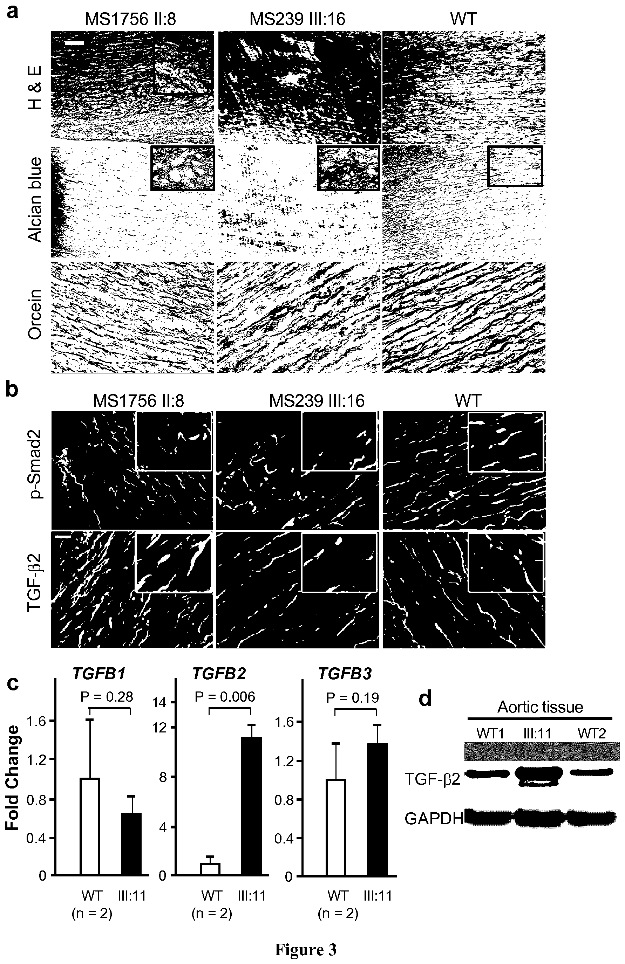

**Figure 3****.** Aortic pathology and assessment of TGF-β signaling in patients with *TGFB2* mutations. **(a)** Histology of aortic media from a dissection case (left column) with a *TGFB2* mutation resulting in p.Glu102* (II:8, MS1756), an aneurysm case (middle column) with a *TGFB2* mutation resulting in p.Cys229* (III:16, MS239 family), and a control (right column). Hematoxylin-eosin (H&E) staining displays disorganization of the aortic media with loss of SMCs and alcian blue reveals proteoglycan accumulation (blue). Note the loss, disarray and fragmentation of elastin fibers versus control with orcein staining. The white scale bars in the upper left pictures are 100µm. (b) Immunohistofluorescent staining for the same patients and control samples. Elastin fibers appear in green (autofluorescence), while nuclei are counter-stained blue. Nuclear pSMAD2 staining (pink, upper panel) is present in the disease tissues and is absent in the control aorta. The lower panel shows the intense immunostaining for TGF-β2 using a polyclonal antibody specific for TGF-β2. Staining for pSMAD2 and TGF-β2 is located in the most disorganized areas of the aortic wall. The white scale bar in the upper left picture is 20 µm. (c) The expression of *TGFB1, TGFB2* and *TGFB3* was assayed by Q-PCR using RNA isolated from the aorta of III:11 from TAA288. The expression of *TGFB2* was increased over 10-fold when compared to aortic tissue isolated from controls aortas. Gene expression levels are standardized to *GAPDH.* The relative expression values were determined via the ΔΔCt method, and assays were performed in triplicate. Data are expressed as mean ± standard error of the mean for pooled experimental results. (d) Immunoblot analysis of the mutant and control aortas showed increased levels of the 47 kD TGF-β2 protein band in the patient's aorta compared with two control aortas using a TGF-β2 specific antibody. Data are expressed as mean ± standard error of the mean for experimental results.

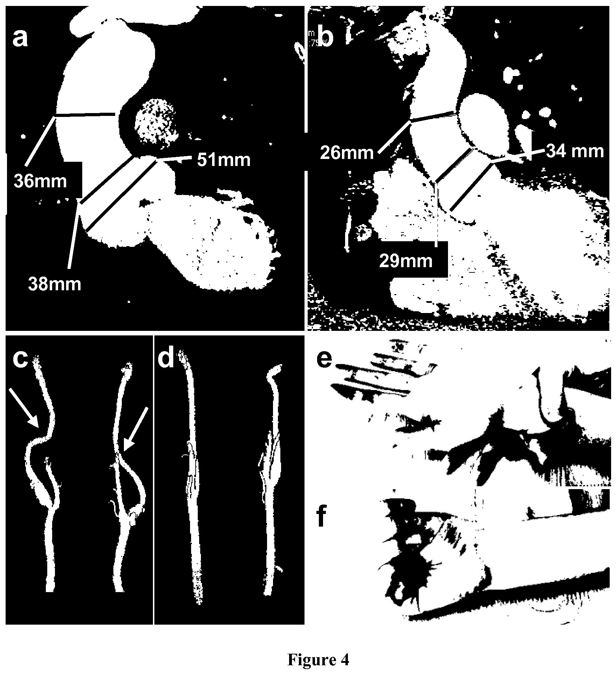

**Figure 4****.** Clinical features associated with *TGFB2* mutations. (a) CT scan imaging of the aorta of patient III:16 from family MS239 demonstrates dilatation predominating at the level of the sinuses of Valsalva (50 mm). (b) CT scan of a normal aorta with a diameter of 34 mm at the level of the sinuses of Valsalva. (c) Three dimensional CT scan from patient MS239, III:16 showing mild tortuosity (arrows) of cerebral arteries compared to normal control (d). Minimal arachnodactyly is evident in individual TAA288 III:8 based on positive wrist (Walker) sign (e) but negative thumb (Steinberg) sign (f).

### EXAMPLE 1: METHODS

The *TGFB2* mRNA reference sequence is available at NCBI (NM_003238.3).

### Whole Genome Linkage Analysis

For the American TAA288 family, genomic DNA from nine family members was analyzed using a 50K GeneChips Hind array from Affymetrix following manufacturer's protocol. Multipoint linkage analyses of the Affymetrix 50K SNP array data was performed with the MERLIN program. An autosomal dominant model for TAAD with a disease-gene frequency of 0.001 was assumed. Two-point linkage analysis with candidate variant status was performed in the families with *TGFB2* mutations. The minor allele frequency of candidate mutation as 0.0001 and penetrance of TAAD as 0.90 were assumed. Log of odds (LOD) scores were calculated with MLINK program of the computer software FASTLINK, version 3.P.²³

For the French MS239 family, genomic DNA from 18 members were analyzed with 1056 microsatellites (deCODE high-density marker set) in multiplex reactions with fluorescently-labeled primers²⁴. All related family members were given an affected, unaffected or unknown status as provided by referring clinicians and in agreement with clinical data. Parametric linkage analyses were performed with the following parameters: (1) autosomal dominant transmission of the disease trait, (2) reduced penetrance of 0.9 for heterozygotes, and (3) frequency of the disease allele of 0.001%. We used Pedcheck to detect Mendelian inheritance errors. SuperLink v1.5 and SimWalk v2.91 programs were used to compute two-point and multipoint LOD scores²⁵⁻²⁷. All these programs were run with the easyLinkage Plus v5.00 package²⁸.

### Exome Sequencing

Genomic DNA was extracted from peripheral blood lymphocytes using standard protocols. Two micrograms of DNA from two affected individuals in family TAA288 were used for construction of the shotgun sequencing library as described previously using adaptors for paired-end sequencing.^{29,30} Exome sequences were captured by SeqCap EZ version 2.0 (Roche) and recovered according to manufacturer's directions. Enriched libraries were then sequenced on a HiSeq using manufacturer protocols. Reads were mapped to the reference human genome (UCSC hg19) with BWA (Burrows-Wheeler Aligner)³¹, and variant detection and genotyping are performed using the UnifiedGenotyper (UG) tool from GATK.³². Single nucleotide variants (SNVs) and indels were filtered to >8X and quality >30. Annotation of variants was performed using the SeattleSeq server (http://gvs.gs.washington.edu/SeattleSeqAnnotation/). The identified variants were then filtered against exome data from non-affected control individuals for indel and SNV calls to identify novel non-synonymous, splice, nonsense or indel variants in affected individuals. These variants were considered as a candidate mutation.

DNA samples from 3 affected and one unaffected individuals in family MS239 were sequenced by Integragen S.A. (Evry, France). The adapter-ligated library was prepared from 3 µg of DNA with the "Paired-End kit PE Sample Prep" (Illumina). DNAs were subjected to the exome capture procedure using the Agilent "SureSelect Human All Exon Kit" (V3, 50Mb) according to manufacturer's protocols. Paired-end sequencing was performed on an Illumina HiSEQ2000 and the short reads (75 bp) were aligned to the Human Genome (UCSC hg19) using CASAVA 1.8. Image analysis and base calling were performed using Illumina Real Time Analysis (RTA) Pipeline version 1.14 with default parameters. The alignment algorithm used was ELANDv2. SNVs and indels were filtered to >8X and quality >30. Variation annotation was performed with the in-house pipeline. Identified variants were then filtered against a further in-house 1500 exome data set to identify novel heterozygous variants that were present in the 3 affected subjects and absent in their healthy relative. These variants were considered as candidate mutations.

### DNA Sequencing

Bidirectional Sanger DNA sequencing assays were performed using primers designed 60-120 base pairs from the variants or intron-exon boundaries to confirm candidate variants or sequence candidate genes on probands from unrelated TAAD familes (Supplementary Table 2). Polymerase chain reaction (PCR) amplifications were carried out using HotStar Taq™ DNA polymerase (Qiagen Inc.Valencia, CA). PCR products were treated with EXOSAP-IT (Affymetrix, Inc. OH) to digest the primers and followed with sequencing PCR using the BigDye™ sequencing reaction mix (Applied Biosystems, CA). The sequencing PCR products were purified using the BigDye XTerminator kit (Applied Biosystems, CA) and then loaded on an ABI3730x1 sequencing instrument using the Rapid36 run module or on an ABI3500xLDx sequencing instrument.. DNA sequencing results were analyzed using the Mutation Surveyor software (SoftGenetics, PA) or SeqScape v2.7 (Applied Biosystem, CA).

### Allele-Specific TGFB2 mRNA Quantification

Allele specific ***TGFB2*** mRNA quantification was performed on mRNA extracted from ascending aortic tissues from patients or control using a TRIzol RNA extraction kit (Life Technologies, Grand Island, NY) and cDNAs were synthesized using MMLV-RT kit (Life Technologies) and random hexamer according to the manufacturer's protocol³³. RT-PCR was performed using primers flanking *TGFB2* mutation and a fluorescently labeled primers ³⁴. The PCR products were assayed using an ABI3730x1 sequencing instrument using the Rapid36 run module and analyzed by GeneMap4.0 software (Life Technologies). The ratio of allele specific expression of *TGFB2* mRNA was obtained based on the ratio of peak areas corresponded to wildtype and mutant alleles.

### Quantitative PCR Analysis

Total cellular RNA was extracted from SMCs, fibroblast cells, or aortic specimens using TRI reagent according to the manufacturer's protocol (Sigma-Aldrich, MO, USA). Q-PCR assays were carried out using pre-designed TaqMan reagents from Life Technologies following the manufacturer's protocols, and were performed on a ABI Prism 7000 Sequence Detection System (Life Technologies, CA, USA).

### Histology Analysis

Aneurysmal ascending aortic and dissection specimens were collected during aortic surgery (Bichat University Hospital). Normal thoracic aorta was obtained from a normal organ transplant donor with the authorization of the French Biomedicine Agency. Aneurysmal tissue was sampled in the outer curvature, the most dilated part of the ascending aorta. The aortic specimens were fixed in 4% (v/v) buffered paraformaldehyde for 48 h at room temperature, embedded in paraffin, and serial sections (5 µm thickness) were obtained from each specimen. The samples were histologically examined after Hematoxylin-Eosin, Alcian Blue (for visualization of proteoglycans) and orcein (for visualization of elastic fibres) staining. Immunohistofluorescence studies were performed as previously described²⁰ using the following primary antibodies: mouse monoclonal anti-TGFβ2 that does not recognize TGFβ1 or TGFβ3 (ab36495 from Abcam, Paris, France), rabbit monoclonal anti pSmad2 (Santa Cruz Biotechnology Inc., CA, USA). Secondary antibodies used were Alexa Fluor 555 anti mouse and anti-rabbit (Invitrogen, Life Technologies SAS, Saint Aubin, France). Nuclei were counter-stained by Hoechst staining (Invitrogen, Life Technologies SAS, Saint Aubin, France).

### Protein Analysis

To isolate proteins from the whole tissue, a piece of cryopreserved tissue (10mg or less) was placed in protein extraction buffer (30mM Hepes, 2.5mM EGTA, 2.5mM EDTA, 20mM KC1, 40mM β-glycerophosphate, 40mM NaF, 4mM NaPPi, 1 mM PMSF, 10 mM NaF, 1 mM Na₃VO₄, 10 ug/ml aprotinin,10 ug/ml leupeptin and 10 ug/ml pepstatin) and homogenized on the Polytron PT-10-35 homogenizer 3 times for 15 seconds each. Samples were then centrifuged, and the pellet fraction discarded. To isolate proteins from SMCs, cells from one patient (III:11) and two normal controls were washed twice with ice-cold PBS and lysed in RIPA buffer (50 mM of Tris, pH 7.5, 150 mM of NaCl, 1% NP-40, 0.5% sodium deoxycholate and 0.1% SDS) supplemented with protease inhibitor cocktail (Sigma) and phosphatase inhibitor cocktail (Sigma). Protein concentration was determined by the Bio-Rad protein assay.

Total lysates from either cells or tissues (10 µg) were separated by SDS-PAGE with 12.5% Tris-HCl gel (Ready Gel, Bio-Rad, Hercules, CA, USA), followed by transfer to polyvinylidene difluoride membranes (Immobilon-P, Millipore, Bedford, MA, USA). Membranes were incubated in blocking buffer (5% nonfat milk in T-PBS) for 1 h and immunoblotted with primary antibody (polyclonal anti-TGF-β2 from R&D System) diluted in 5% BSA (Sigma-Aldrich, MO, USA). Membranes were probed with horseradish peroxidase-conjugated secondary antibody (Jackson ImmunoResearch Laboratories, West Grove, PA, USA). Western blots were visualized by the enhanced chemiluminescence technique (Amersham ECL Western Blotting Detection Reagents, GE Healthcare, Piscataway, NJ, USA). Densitometry was performed using ImageJ software. Each band was quantified three independent times, and results shown were pooled from at least two separate immunoblots.

### Statistical Analysis of RNA and Protein Expression

All values are expressed as means ± standard error of the mean (standard deviation divided by the square root of the number of separate experimental replicates). Statistical differences between *TGFB2* and control RNA or protein expression were analyzed by a Student's t-test. Differences were considered statistically significant at values of P <0.05. Data shown was pooled from at least two experimental replicates.

### EXAMPLE 2: TGFB2 loss of function mutations cause familial thoracic aortic aneurysms and acute aortic dissections associated with mild systemic features of the Marfan syndrome

Thoracic aortic aneurysms can lead to acute aortic dissections or ruptures, and death due to these complications is common¹. A number of genetic syndromes predispose to thoracic aortic disease, including Marfan syndrome (MFS, MIM 154700), Loeys-Dietz syndrome (LDS, MIM 609192, 608967, 610168, 610380) and Aneurysms-Osteoarthritis Syndrome (AOS, MIM 613795)²⁻⁴. Systemic complications are shared among these syndromes, including skeletal, craniofacial and skin manifestations. LDS is caused by mutations in the genes encoding the TGF-β receptors type I and II (*TGFBR1* and *TGFBR2,* respectively), which bind TGF-β and initiate cellular signaling. AOS results from mutations in *SMAD3* (SMAD family members 3), which encodes a protein critical for cellular signaling downstream of the TGF-β receptors after ligand binding. Despite the fact that the majority of mutations in these genes are predicted to disrupt proper function of these signaling proteins, diseased aortic tissue from these patients show increased nuclear phosphorylated SMAD2 (pSMAD2) in aortic smooth muscle cells (SMCs), suggesting increased TGF-β cellular signaling^{3,4}. Although MFS is caused by mutations in *FBN1*, encoding an extracellular matrix protein called fibillin-1, excessive TGF-β signaling is also evident in aortic tissue from MFS patients and mouse models^{5,6}.

We sought to identify the defective gene in two unrelated large American (TAA288) and French (MS239) families with autosomal dominant thoracic aortic aneurysms and aortic dissections with decreased penetrance, along with intracranial aneurysms (ICAs) and subarachnoid hemorrhages (SAHs), after sequencing of the known genes failed to identify causative mutations **(****Fig. 1a****)**⁷. Genome wide linkage analysis followed by exome sequencing was pursued to identify the defective gene in these families. In TAA288, linkage analysis using 50K GeneChips Hind array from Affymetrix was done using samples from 9 family members and multipoint log10 of odds (LOD) scores greater than 2.0 were obtained for a locus on chromosome 1 (**Fig. 1b**). Similar analysis of the MS239 family using 1056 microsatellites was done using samples from 18 family members and LOD scores greater than 1.4 were obtained on chromosomes 1, 4, 12, and 20 (**Fig. 1c**)**.** A locus at chromosome 1q41 was shared between the two families. Exome sequencing was pursued in both families using DNA from two distantly related affected individuals (1/16 coefficient of relatedness) from TAA288 and 3 affected and 1 unaffected family members from MS239 (circled individuals in **Fig. 1a**). The sequencing results were filtered based on novel, heterozygous rare variants shared between affected relatives within a family that were non-synonymous, nonsense, and insertion/deletion variants. This filtering strategy identified 16 rare variants in TAA288 and 5 rare variants in MS239 (**Supplementary Table 1**). Rare variants in *TGFB2* were present in both families and *TGFB2* fell within the 1q41 locus identified for the causative gene in both families (**Fig. 1b** and **1c**). The *TGFB2* rare variant in TAA288 was a 5 base pair deletion (c.1021_1025delTACAA) in exon 6 of the gene, leading to a frameshift in the translation of the transcript and a premature stop codon (p.Tyr341Cysfs*25; **Fig. 1d**). This variant had a two point linkage LOD score of 3.3 (theta = 0.0) and was not present in 3795 control exomes based on bioinformatic analysis and visual inspection of the sequencing data. The *TGFB2* variant in MS239 was a nonsense variant in exon 4 (p.Cys229*). Complete segregation with thoracic aortic disease was found in the family with a LOD score of 4.4 (theta = 0.0). This nonsense rare variant was not present in over 10,000 chromosomes (http://evs.gs.washington.edu/EVS/). In fact, there are no *TGFB2* rare variants predicted to disrupt translation of the protein in the database (**Fig. 1d**). Thus, these data provide strong evidence that the identified *TGFB2* mutations are responsible for the inherited vascular disease in both families.

To determine the frequency and spectrum of *TGFB2* mutations in individuals with thoracic aortic disease, the 7 exons and flanking intronic regions were sequenced using DNA from French probands (62 familial cases and 74 sporadic cases from a Marfan referral clinic) and American probands (214 affected probands in families with two or more members with thoracic aortic disease, including 30 families who also had members with intracranial aneurysms, and 57 sporadic thoracic aortic disease cases)⁷(**Supplementary Table 2**). Two further mutations were identified in the French familial cases: a nonsense p.Glu102* in MS1756 and frameshift duplication (c.873_888dup) leading to p.Asn297* in MS211; additional family members were not available for analysis. Therefore, *TGFB2* mutations were identified in 2 out of 276 probands with familial thoracic aortic disease. Genome SNP array data on 898 patients with thoracic aortic disease, including 88 patients with inherited thoracic aortic disease, were analyzed for genomic rearrangements using CNVPartition and PennCNV and the B allele frequencies and logR ratio files visually inspected. No deletions or duplications involving *TGFB2* were identified⁸.

The *TGFB2* nonsense mutations and the frameshift duplication in exon 5 are predicted to lead to degradation of the message and thus cause haploinsufficiency for *TGFB2.* The mutation identified in TAA288, p.Tyr341Cysfs*25, is a deletion of 5 base pairs in exon 6, causing a frameshift in protein translation and addition of 25 aberrant amino acids. Since the nonsense codon that terminates protein translation occurs in exon 7, it is not expected to cause nonsense mediated decay⁹. Analysis of the *TGFB2* transcript confirmed that similar levels of both mutant and wildtype transcript were present in RNA harvested from aortic tissue and fibroblasts from affected family members (III:11 and IV:1; **Fig. 2a****; Supplementary Table** 2). The TGF-β family members are synthesized as peptides that form disulfide cross-linked homodimers (proprotein), which are cleaved by furin immediately prior to secretion. *TGFB2* expression levels were similar in SMCs and dermal fibroblasts explanted from affected family members in TAA288 and control cells (**Fig. 2b**), but immunoblot analysis of the TGF-β2 proprotein in cellular lysates from these cells showed reduced TGF-β2 proprotein levels in the mutant cells compared with the wildtype cells and no evidence of the truncated protein (**Fig.** 2c). These data suggest that although the deleted transcript is expressed, the mutant protein is rapidly degraded by an endoplasmic reticulum-associated degradation pathway, resulting in decreased cellular levels of wildtype TGF-β2.

The aortic pathology associated with *TGFB2* mutations was assessed using aortic tissue from patients MS239 III:16 and MS1756 II:8. The aortic pathology typical for thoracic aortic disease was found, which is characterized by fragmentation and loss of elastin fibers and accumulation of proteoglycans in the tunica media (**Fig. 3a**). Assessment of nuclear pSMAD2 in medial SMCs from the *TGFB2* mutant aortas showed increased staining when compared with control aortas (**Fig. 3b**). *TGFB1*, *TGFB2* and *TGFB3* expression levels were assessed by Q-PCR using aortic tissue RNA from III:11 in family TAA288 and two matched controls (**Fig. 3c**). Surprisingly, only *TGFB2* expression was increased in the aortic tissue (**Fig. 2b**). Immunoblot analysis of protein lysates from these same aortas demonstrated a corresponding increase in TGF-β2 proprotein levels in the mutant aorta (**Fig. 3d**). Immunostaining of TGF-β2 in aortic tissue from patients MS239 III:16 and MS1756 II:8 also showed increased intensity of the signal in patients' aortas compared with control aorta (**Fig. 3b**). Thus, the aortas from patients with *TGFB2* mutations show enhanced TGF-β signaling associated with a paradoxical increase in *TGFB2* expression and TGF-β2 protein levels.

The clinical features in *TGFB2* mutation carriers were assessed and found to be similar to other syndromes causing thoracic aortic disease. The median age at aortic disease presentation was 35 years, with the majority of affected family members presenting with aneurysms at the level of the sinuses of Valsalva **(****Fig. 4a****,b; Table 1; Supplementary Table 3).** A 60 year old male underwent surgical repair after his aneurysm enlarged from 47 to 50 mm over six years, and a 35 year old male underwent repair with an aortic root diameter of 54 mm. Aortic dissection was documented in an obligate mutation carrier in MS239 (III:12, female 56 years), and in the probands of MS1756 (II:8, male, 47 years) and MS211 (III:14, male, 31 years). Three women who were obligate carriers presented with cerebrovascular disease; TAA288-II:1 had a ruptured anterior communicating artery aneurysm at age 54 years, and TAAII:3 and MS239-III:2 died of SAH presumably due to ruptured ICAs. Fusiform dilatation and tortuosity of cerebrovascular arteries were present in mutation carriers (**Fig. 4c****,d**). Additional cardiovascular features included interventricular septal defect present in an obligate carrier from MS239 (III:12), mitral regurgitation requiring surgical repair at the age of 43 years in IV:2 from MS239, and dilated cardiomyopathy prior to aortic root aneurysm formation in III:3 from TAA288. Inguinal hernias, typically bilateral and requiring surgical repair, were common in mutation carriers, including women. Skeletal features, joint laxity, and skin striae similar to those observed in MFS patients were found in some individuals with *TGFB2* mutations but were insufficient in any individual to meet the diagnostic criteria of MFS (**Table 1,** **Fig. 4e****,f**)¹⁰. Ectopia lentis was absent in these families. The affected family members examined by clinical geneticists (13 out of 19 mutation carriers) did not have a particular facial appearance or the craniofacial or skin findings observed in LDS patients. Prominent degenerative joint disease described in AOS patients was not present, although age- and trauma-related osteoarthritis was present.

We report a new etiology for familial thoracic aortic disease due to loss of function mutations in the *TGFB2* gene. Aortic dilatation occurs at the level of the sinuses of Valsalva, similar to what is observed with mutations in *FBN1*, *TGFBR1*, *TGFBR2*, and *SMAD3.* The occurrence of ICAs and SAHs in three obligate carriers is most likely a manifestation of the *TGFB2* mutation since similar cerebrovascular disease occurs in individuals with *TGFBR1*, *TGFBR2,* or *SMAD3* mutations. Lastly, some systemic features overlapping with Marfan syndrome are also observed, as is the case with other syndromic forms of thoracic aortic disease (**Table 1**) ¹¹. It should be underscored that no aortic dissections occurred younger than 31 years of age or with minimal aortic enlargement.

Mice with specific genetic disruption of each of the genes for the three isoforms of TGF-β (*Tgfbl*, *Tgfb2* and *Tgfb3*) have discrete phenotypes, indicating a distinct role for each of the TGF-β isoforms. *Tgfb2*^{*-*/*-*} mice die shortly after birth and have ascending aortas that are comparatively small and thin walled and other development defects involving the heart, lung, craniofacial, limb, spinal column, eye, inner ear, and urogenital systems¹². The developmental processes disrupted by loss of TGF-β2 include epithelial to mesenchymal transformation (EMT) and differentiation of neural crest-derived components. These mice also show dysmorphic calvaria, extensive cleft palate, defective mandibles, and retrognathia; only retrognathia was observed in one *TGFB2* mutation carrier. Based on our findings, haploinsufficiency of TGF-β2 does not disrupt cardiovascular development, suggesting that reduction of TGF-β2 level does not disrupt proper migration or homing of cells during development. Rather, decreased levels of TGF-β2 predispose to adult-onset vascular diseases involving the sinuses of Valsalva and the cerebrovascular arteries. SMCs in these two vascular beds are derived from two different origins, the secondary heart field and neural crest cells, respectively¹³. Thus, the clinical features suggest that decreased TGF-β2 levels lead to a defect in differentiation, proliferation or viability of cells in these arteries. The differentiation of neural crest cells into vascular smooth muscle cells is dependent on TGF-β signaling and secondary heart field EMT is specifically TGF-β2 dependent^{12,14}, and decreased levels of TGF-β2 may disrupt proper differentiation of SMCs in these arteries. Relevant to this speculation, it is notable that SMCs explanted from patients with *TGFBR2* mutations are relatively de-differentiated in culture when compared to control SMCs, and fail to differentiate to the same extent as control SMCs when exposed to TGF-β1¹⁵.

Our results provide evidence that haploinsufficiency of *TGFB2* is an initiating step in the pathogenic events leading to thoracic aortic disease. A subset of the *SMAD3* mutations are frameshift mutations that are predicted to cause *SMAD3* haploinsufficiency^{16,17}. The missense mutations in *TGFBR1* and *TGFBR2* have either been shown or are predicted to disrupt kinase function, which is critical for propagating TGF-β signaling^{4, 15, 16, 18, 19}. Despite the fact that these mutations are predicted to decrease TGF-β signaling, these defective genes trigger poorly understood signaling events that lead to increased nuclear pSMAD2 immunostaining in diseased tissue. Immunostaining for pSMAD2 is also increased in aortas of thoracic aortic disease patients without mutations in these genes⁵. One event contributing to increased pSMAD2 staining may be epigenetic modifications of the promoter of *SMAD2*, resulting in constitutive activation of this signaling protein²⁰. Another possible contributing event is that the relative resistance of the SMCs to TGF-β signaling due to the gene mutations may paradoxically increase signaling through other TGF-β signaling proteins. It is notable that deletion of *Tgfbr2* in mouse cranial neural crest cells leads to elevated expression of TGF-β2 and TGF-β type III receptor and activation of SMAD-independent cellular pathways²¹. Our study identified a similar increase in TGF-β2 levels in the diseased tissue in response to *TGFB2* haploinsufficiency that is not present in explanted SMCs and fibroblasts. Although our data support the conclusion that the initiating event leading to vascular diseases due to mutations in *TGFB2* is decreased TGF-β2 levels, further work is warranted to unravel the pathways that are secondarily altered to cause a paradoxical increase in *TGFB2* expression and increased pSmad2 signaling in diseased aortic tissue.

**Table 1 Comparison of the clinical features observed in patients with TGFB2 mutations and patients with TGFBR2, FBN1, or SMAD3 mutations.**

| **Feature** | **Frequency, no. individuals/total (%)^{a}** | | | |
|---|---|---|---|---|
| | ***TGFB2*** | ***TGFBR2*** | ***FBN1*** | ***SMAD3*** |
| Cardiovascular: | | | | |
| Aortic root aneurysm | 14/19 (74) | 54/69 (78) | 178/226 (79) | 28/39 (72) |
| Aortic dissection | 3/23 (13) | 10/71 (14) | 23/243 (10) | 13/39 (33) |
| Cerebrovascular disease | 3/10 (30) | 0^{b} | 0^{b} | 6/16 (38) |
| Arterial tortuosity | 3/5 (60) | 5/25 (20) | NA | 8/16 (50)^{c} |
| Mitral valve prolapse | 3/19 (16) | 15/66 (21) | 105/232 (45) | 18/36 (50)^{d} |

| Skeletal: | | | | |
|---|---|---|---|---|
| Pectus deformity | 7/16 (44) | 31/69 (45) | 144/243 (59) | 12/33 (36) |
| Arachnodoctyly | 8/13 (62) | 28/70 (41) | 137/240 (57) | 13/33 (39) |
| Spondylolisthesis | 1/7 (14) | 5/47 (11) | 15/223 (7) | 10/26 (38) |
| Scoliosis | 4/15 (27) | 21/68 (31) | 122/240 (51) | 22/36 (61) |
| Flat feet | 11/15 (73) | 8/58 (14) | 108/243 (44) | 30/33 (91) |
| Protrusio acetabularis | 1/8 (12) | 3/47 (6) | 79/221 (36) | 7/20 (35) |
| Joint hyperflexibility | 10/15 (67) | 45/59 (76) | 168/243 (69) | 3/31 (10) |
| High arched palate | 9/15 (60) | 32/67 (48) | 151/243 (62) | 15/28 (54)^{d} |

| Cutaneous: | | | | |
|---|---|---|---|---|
| Striae atrophicae | 8/15 (53) | 24/66 (36) | 139/241 (58) | 17/32 (53) |
| Operated hernia | 6/17(35) | 13/67(19)^{e} | 40/239 (17)^{e} | 17/40(43) |

| Pulmonary: | | | | |
|---|---|---|---|---|
| Pneumothorax | 1/17 (6) | 3/69 (4) | 10/240 (4) | NA |
| Dural ectasia | 3/5 (60) | 4/40 (10) | 64/220 (29) | NA |

| | | | | |
|---|---|---|---|---|
| ^{a} The frequency of clinical features associated with *TGFBR2*, *FBN1* and *SMAD3* are based on previous publications ^{11, 17, 22}. Provided are the number of individuals with the feature/total number of individuals assessed for the particular feature (percentage); ^{b} based on medical history (imaging to screen for asymptomatic vascular disease not done); ^{c} number of patients with tortuosity of the cerebral arteries; ^{d} designated in publication as abnormal palate; ^{e} number of individuals with recurrent hernias; NA- data not available. | | | | |

**Supplementary Table 1 Rare nonsynonymous, nonsense, and indel variants identified by exome sequencing that are segregated with deseases in families**

| Gene | Location | TAAD family ID | Nucleotide change | aminoAcids | polyPhen | Present in controlsa |
|---|---|---|---|---|---|---|
| TGFB2 | chr1:218610772 | TAA288 | GTACAA/G | p.Tyr341Cysfs*25d | NA | 0/3795b |
| FAM5C | chr1:190068100 | TAA288 | A/G | Leu450Pro | benign | 0/1525 |
| DISP1 | chr1 :223177116 | TAA288 | G/A | Val793Met | probably-damaging | 1/1536 |
| ZNF488 | chr10:48370770 | TAA288 | C/G | Pro80Ala | probably-damaging | 0/1329 |
| COL4A2 | chr13:111156311 | TAA288 | T/C | Met1419Thr | probably-damaging | 3/1157 |
| UIMC1 | chr5:176332446 | TAA288 | T/G | Gln666Pro | probably-damaging | 3/1537 |
| BDP1 | chr5:70798515 | TAA288 | C/T | Pro713 Leu | probably-damaging | 1/1537 |
| GPT | chr8:145730072 | TAA288 | G/A | Arg83His | probably-damaging | 0/1534 |
| STK32A | chr5:146741152 | TAA288 | C/T | Thr212Met | possibly-damaging | 0/1529 |
| LPIN2 | chr18:2922148 | TAA288 | C/T | Asp742Asn | possibly damaging | 0/1490 |
| COL4A2 | chrl3:111121570 | TAA288 | A/G | Lys701Arg | benign | 3/1537 |
| DGCR8 | chr22:20073742 | TAA288 | C/A | Leu86Ile | benign | 2/1537 |
| NIPAL4 | chr5:156899672 | TAA288 | G/A | Val369Ile | benign | 1/1537 |
| FAM83H | chr8:144812521 | TAA288 | G/A | Pro78Ser | benign | 0/1537 |
| CABYR | chr18:21736772 | TAA288 | C/G | Ser436Cys | NA | 1/1535 |
| DYTN | chr2:207527977 | TAA288 | T/TCC | p.Glu428Glyfs*59 | NA | 7/1537 |
| TGFB2 | chr1:218607723 | MS239 | C/A | C229X | NA | 0/10750 (EVS)c |
| PARP4 | chr13:25021200 | MS239 | C/T | L1080F | benign | 0/10752 (EVS) |
| USP32 | chr17:58288396 | MS239 | A/G | N801S | benign | 680/10680 (EVS) |
| NBPF10 | chr1:145298168 | MS239 | A/G | A194T | possibly damaging | 0/3230 (EVS) |
| TMEM206 | chr1:212560248 | MS239 | C/T | R171C | possibly damaging | 15/10758 (EVS) |

| | | | | | | |
|---|---|---|---|---|---|---|
| ^{a}Rare variants present in the number of control chromosomes. ^{b}control data were obtained from in-house 1500 exome data set. ^{c}control data were obtained from the EVS database (http://evs.gs.washington.edu/EVS/). ^{d} the only exome rare variant identified in family TAA288 that segregated with thoracic aortic aneurysms and aortic dissections when additional affected family members were assessed. | | | | | | |

**Supplementary Table 2 Primers used for TGFB2 sequencing and mRNA quantification**

| Primers | Primer sequence (5' → 3') |
|---|---|
| RT-PCR forward primer | TCGAGGACCTGACACGAGGGTGGAAATGGATACACGA (SEQ ID NO :2) |
| RT-PCR reward primer | GGAGAAGCAGATGCTTCTGG (SEQ ID NO :3) |
| Exon 1 forward primer | CCAAACAACTCTCCTTGATCTA (SEQ ID NO :4) |
| Exon 2 forward primer | CCCTTATGGTTTCTTGGGAT (SEQ ID NO :5) |
| Exon 3 forward primer | GTGACCATGCAATTGAGATG (SEQ ID NO :6) |
| Exon 4 forward primer | GAATGGCTTCACCATAAAGG (SEQ ID NO :7) |
| Exon 5 forward primer | CAGCTGATGCTGCTTTGGTG (SEQ ID NO :8) |
| Exon 6 forward primer | cACTTGaGaTTacaaTaaaGCC (SEQ ID NO :9) |
| Exon 7 forward primer | GCCTACTCAGTGCTGTGAC (SEQ ID NO :10) |
| Exon 1 reward primer | AAGGGCGATCCCGGGAGCTG (SEQ ID NO :11) |
| Exon 2 reward primer | GTTCaaGGTTGCaGTGaGCT (SEQ ID NO:12) |
| Exon 3 reward primer | CAAGTTTTGGTTACCTGC (SEQ ID NO :13) |
| Exon 4 reward primer | CAACCATATCACTGTCCAAATG (SEQ ID NO 14) |
| Exon 5 reward primer | TGCACTCTATCTGGCCTT (SEQ ID NO :15) |
| Exon 6 reward primer | CCAGTACCCATACATGTG (SEQ ID NO: 16) |
| Exon 7 reward primer | ACTGATGAACCAAGGCTCT (SEQ ID No :17) |

**Supplementary Table 3 Clinical data of individuals with TGFB2 mutations**

| | | | | | TAA288 | | | | | | | | | MS 239 | | | | MS1 758 | MS 211 |
|---|---|---|---|---|---|---|---|---|---|---|---|---|---|---|---|---|---|---|---|
| Features | III :2 | III :3 | III :4 | III :5 | III :8 | III :9 | III: 11 | III: 12 | IV: 1 | IV: 2 | IV :3 | III: 16 | IV: 2 | IV:1 2 | IV: 20 | IV: 22 | V:5 | II:8 | III:1 4 |
| Sex | F | M | F | M | F | M | M | M | M | M | M | M | M | F | F | M | M | M | M |
| Age (years) | 50 | d. 41 | 46 | 47 | 32 | 43 | 42 | 39 | 22 | 18 | 10 | 59 | 56 | 41 | 39 | 27 | 17 | 50 | 33 |
| Ht (cm) | 17 | 19 | 16 | 17 | 17 | 19 | 19 | 20 | 18 | 18 | 15 | 18 | 19 | 182 | 175 | 179 | 187 | 165 | 179 |
| | 8 | 1 | 8 | 3 | 3 | 8 | 0 | 1 | 0 | 5 | 7 | 2 | 0 | | | | | | |
| Wt (kg) | 64 | 84 | 10 5 | 71 | 55 | 12 2 | 94 | 89 | 67 | 73 | 44 | 84 | 90 | 82 | 57 | 60 | 89 | 60 | 75 |
| Age at diagnosis (years) | | 41 | | 40 | 25 | 36 | 35 | 32 | 18 | 17 | 5 | 53 | 45 | 36 | 32 | 27 | | 47 | 31 |
| Age at surgery (years) | | | | | | | 35 | | | | | 60 | 43 | | | | | 47 | 31 |
| Reason for | | | | | | | TA | | | | | TA | M | | | | | TAD | TA |
| surgery | | | | | | | A | | | | | A | VP | | | | | | D |
| Annulus (cm) | . | . | N1 * | . | . | . | . | . | . | . | . | 2.6 | 2.8 | 2.5 | 2.2 | 2.4 | 2.4 | | |
| Valsalva (cm) | 2. 6 | 8^{*} | N1 * | 4. 7 | 3. 4-3. 5 | 4.7 (C T) | 5.4 (C T) | 4.1 | 4.1 | 3.4 | 2. 9 | 5 | 4.8 | 4.1 | 4.6 | 3.6 | 3.7 | | |
| Sinotubula r junction (cm) | . | . | N1 * | . | . | . | . | . | . | . | 2. 0 | 3.4 | 3.5 | 3.2 | 2.6 | 2.6 | 3 | | |
| Ascending aorta (cm) | . | . | N1 * | . | . | . | . | . | . | . | 1. 7 | 3.5 | 3.5 | 3.2 | 2.3 | 2.5 | 2.8 | | |
| Z score, sinuses of Valsalva | . | . | . | . | 2. 7 | . | . | . | 5.1 | 2.4 | 2. 1 | 4.2 | 3.4 | 2.24 | 7.2 | 2.7 5 | 1.3 | | |
| LVEDD (cm) | . | . | . | . | . | . | . | . | . | . | | 5.7 | 5.4 | 6.0 | 4.7 | 5.0 | 6.1 | 4.9 | 5.9 |
| Arterial tortuosity | . | . | . | . | no | . | . | . | . | . | | yes | yes | . | . | . | . | yes | no |
| Intracrania 1 aneurysm | no | . | no | . | no | . | . | . | no | no | | no | no. | . | . | . | . | . | . |
| Other cardiac disease | | DC M | | | | | | | M VP | M VP | | | M VP | | | | | | |
| Pectus deformity | no | . | . | . | no | no | miL d | mil d | no | no | mi 1d | mil d | yes | no | yes | no | no | yes | no |
| Arm span to height ratio | 0. 93 | . | . | . | 1. 02 | | 1.0 3 | . | 0.9 4 | 1.0 2 | 0. 99 | 1.0 2 | 1.0 7 | 1.04 | 0.9 6 | 1.0 1 | 1.0 1 | 1.04 | 1.02 |
| Wrist sign | no | . | . | . | ye s | | yes | . | no | no | . | no | no | yes | yes | no | no | yes | yes |
| Thumb sign | no | . | . | . | no | | yes | . | no | yes | . | no | yes | yes | yes | no | no | yes | yes |
| Spondyloli sthesis | . | . | . | . | . | . | . | . | . | . | . | no | yes | no | no | no | no | no | . |
| Scoliosis | ye s | . | . | . | ye s | . | no | no | mil d | no | no | no | no | mild | no | no | no | no | no |
| Flat feet | ye s | . | . | . | no | . | yes | yes | yes | yes | no | yes | yes | no | yes | yes | yes | yes | no |
| Protrusio acetabularis | . | . | . | . | . | . | . | . | . | . | . | no | yes | no | no | no | no | no | no |
| Joint hyperflexi bility | ye s | . | . | . | ye s | . | no | yes | yes | yes | no | no | no | yes | yes | no | yes | yes | yes |
| High arched palate | ye s | . | . | . | ye s | . | no | yes | yes | yes | ye s | yes | yes | no | yes | no | no | no | no |
| Striae atrophicae | no | . | . | . | no | . | yes | yes | yes | yes | no | yes | yes | no | no | yes | yes | no | no |
| Operated hernia | no | ye s | . | ye s | no | . | yes | no | yes | no | no | no | no | yes | no | no | no | no | yes |
| Cutis laxa | no | | | | no | | | | no | no | no | no | no | no | no | no | no | no | no |
| Pneumoth orax | no | . | . | no | no | no | no | no | no | no | no | no | no | yes | no | no | no | no | no |
| Dural ectasia | . | . | . | . | . | . | . | . | . | . | . | no | yes | yes | . | no | . | yes | . |
| Emphysema | no | no | . | no | no | no | no | no | no | no | no | no | yes | . | . | . | . | yes | . |
| Phlebitis | no | no | . | no | no | no | no | no | no | no | no | yes | yes | no | no | no | no | no | no |
| Lens | no | . | . | . | no | . | . | . | no | no | no | no | no | no | no | no | min | no | no |
| dislocation | | | | | | | | | | | | | | | | | or^{†} | | |
| Flat cornea | . | . | . | . | . | . | . | . | . | . | . | no | yes | no | no | no | yes | no | no |
| Dolichoce phaly | no | . | . | . | no | . | . | . | no | no | no | no | no | no | no | no | no | no | no |
| Malar hypoplasia | no | . | . | . | no | . | . | . | no | no | no | no | no | no | no | no | no | no | no |
| Down-slanting palpebral fissures | no | . | . | . | no | . | . | . | no | no | no | no | no | no | no | no | no | no | no |
| Retrognathia | no | . | . | . | no | . | . | . | no | yes | no | no | no | yes | no | no | no | no | no |
| Enophthalmos | no | . | . | . | no | . | . | . | no | no | no | no | no | no | no | no | no | no | no |
| Hypertelorism | no | . | . | . | no | . | . | . | no | no | no | no | no | no | no | no | no | no | no |
| Cleft palate or abnormal uvula | no | . | . | . | no | . | . | . | no | no | no | no | no | no | no | no | no | no | no |
| Craniosyn ostosis | no | . | . | . | no | . | . | . | no | no | no | no | no | no | no | no | no | no | no |

| | | | | | | | | | | | | | | | | | | | |
|---|---|---|---|---|---|---|---|---|---|---|---|---|---|---|---|---|---|---|---|
| Ht : height; Wt: weight; TAA: thoracic aortic aneurysm; TAD: thoracic aortic dissection; "." : data not available; "*" : verbal report; blank: not applicable; N1 : normal; aortic measurements were obtained by echocardiography unless otherwise noted; MR: obtained by MR imaging; CT: obtained by CT imaging;; DCM: dilated cardiomyopathy; MVP: mitral valve prolapse; ^{†}individual noted as having minor ectopia lentis by French ophthalmologists, which is not recognized as lens dislocation in the USA. | | | | | | | | | | | | | | | | | | | |

### REFERENCES:

Throughout this application, various references describe the state of the art to which this invention pertains.
1. Hiratzka,L.F. et al. 2010 ACCF/AHA/AATS/ACR/ASA/SCA/SCAI/SIR/STS/SVM Guidelines for the Diagnosis and Management of Patients With Thoracic Aortic Disease: Executive Summary. A Report of the American College of Cardiology Foundation/American Heart Association Task Force on Practice Guidelines, American Association for Thoracic Surgery, American College of Radiology, American Stroke Association, Society of Cardiovascular Anesthesiologists, Society for Cardiovascular Angiography and Interventions, Society of Interventional Radiology, Society of Thoracic Surgeons, and Society for Vascular Medicine. Circulation 121, e266-369 (2010).
2. Faivre,L. et al. Pathogenic FBN1 mutations in 146 adults not meeting clinical diagnostic criteria for Marfan syndrome: further delineation of type 1 fibrillinopathies and focus on patients with an isolated major criterion. Am. J. Med. Genet. A 149A, 854-860 (2009).
3. Loeys,B.L. et al. Aneurysm syndromes caused by mutations in the TGF-beta receptor. N. Engl. J Med. 355, 788-798 (2006).
4. van de Laar,I.M. et al. Mutations in SMAD3 cause a syndromic form of aortic aneurysms and dissections with early-onset osteoarthritis. Nat. Genet. 43, 121-126 (2011).
5. Gomez,D. et al. Syndromic and non-syndromic aneurysms of the human ascending aorta share activation of the Smad2 pathway. J. Pathol. 218, 131-142 (2009).
6. Habashi,J.P. et al. Losartan, an ANT1 antagonist, prevents aortic aneurysm in a mouse model of Marfan syndrome. Science 312, 117-121 (2006).
7. Regalado,E. et al. Autosomal dominant inheritance of a predisposition to thoracic aortic aneurysms and dissections and intracranial saccular aneurysms. Am. J. Med. Genet. A 155A, 2125-2130 (2011).
8. Prakash,S.K. et al. Rare copy number variants disrupt genes regulating vascular smooth muscle cell adhesion and contractility in sporadic thoracic aortic aneurysms and dissections. Am. J Hum. Genet. 87, 743-756 (2010).
9. Silva,A.L. & Romao,L. The mammalian nonsense-mediated mRNA decay pathway: to decay or not to decay! Which players make the decision? FEBS Lett. 583, 499-505 (2009).
10. Loeys,B.L. et al. The revised Ghent nosology for the Marfan syndrome. J. Med. Genet. 47, 476-485 (2010).
11. Attias,D. et al. Comparison of clinical presentations and outcomes between patients with TGFBR2 and FBN1 mutations in Marfan syndrome and related disorders. Circulation 120, 2541-2549 (2009).
12. Sanford,L.P. et al. TGFbeta2 knockout mice have multiple developmental defects that are non-overlapping with other TGFbeta knockout phenotypes. Development 124, 2659-2670 (1997).
13. Majesky,M.W. Developmental basis of vascular smooth muscle diversity. Arterioscler. Thromb. Vasc. Biol. 27, 1248-1258 (2007).
14. Chen,S. & Lechleider,R.J. Transforming growth factor-beta-induced differentiation of smooth muscle from a neural crest stem cell line. Circ. Res. 94, 1195-1202 (2004).
15. Inamoto,S. et al. TGFBR2 Mutations Alter Smooth Muscle Cell Phenotype and Predispose to Thoracic Aortic Aneurysms and Dissections. Cardiovasc. Res. 88, 520-529 (2010).
16. Regalado,E.S. et al. Exome Sequencing identifies SMAD3 mutations as a cause of familial thoracic aortic aneurysm and dissection with intracranial and other arterial aneurysms. Circ. Res. 109, 680-686 (2011).
17. van de Laar, I.M. et al. Phenotypic spectrum of the SMAD3-related aneurysms-osteoarthritis syndrome. J Med. Genet 49, 47-57 (2012).
18. Mizuguchi,T. et al. Heterozygous TGFBR2 mutations in Marfan syndrome. Nat. Genet. 36, 855-860 (2004).
19. Stheneur,C. et al. Identification of 23 TGFBR2 and 6 TGFBR1 gene mutations and genotype-phenotype investigations in 457 patients with Marfan syndrome type I and II, Loeys-Dietz syndrome and related disorders. Hum. Mutat. 29, E284-E295 (2008).
20. Gomez,D. et al. Epigenetic control of vascular smooth muscle cells in Marfan and non-Marfan thoracic aortic aneurysms. Cardiovasc. Res. 89, 446-456 (2011).
21. Iwata,J. et al. Modulation of noncanonical TGF-beta signaling prevents cleft palate in Tgfbr2 mutant mice. J. Clin. Invest 122, 873-885 (2012).
22. Faivre,L. et al. Effect of mutation type and location on clinical outcome in 1,013 probands with Marfan syndrome or related phenotypes and FBN1 mutations: an international study. Am. J. Hum. Genet. 81, 454-466 (2007).
23. Cottingham,R.W., Jr., Idury,R.M., & Schaffer,A.A. Faster sequential genetic linkage computations. Am. J. Hum. Genet. 53, 252-263 (1993).
24. Kong,A. et al. A high-resolution recombination map of the human genome. Nat. Genet 31, 241-247 (2002).
25. O'Connell,J.R. & Weeks,D.E. PedCheck: a program for identification of genotype incompatibilities in linkage analysis. Am. J. Hum. Genet. 63, 259-266 (1998).
26. Fishelson,M. & Geiger,D. Exact genetic linkage computations for general pedigrees. Bioinformatics. 18 Suppl 1, S 189-S 198 (2002).
27. Weeks,D.E., Sobel,E., O'Connell,J.R., & Lange,K. Computer programs for multilocus haplotyping of general pedigrees. Am. J. Hum. Genet. 56, 1506-1507 (1995).
28. Lindner,T.H. & Hoffmann,K. easyLINKAGE: a PERL script for easy and automated two-/multi-point linkage analyses. Bioinformatics. 21, 405-407 (2005).
29. O'Roak,B.J. et al. Exome sequencing in sporadic autism spectrum disorders identifies severe de novo mutations. Nat. Genet. 43, 585-589 (2011).
30. Ng,S.B. et al. Exome sequencing identifies the cause of a mendelian disorder. Nat. Genet. 42, 30-35 (2010).
31. Li,H. & Durbin,R. Fast and accurate long-read alignment with Burrows-Wheeler transform. Bioinformatics. 26, 589-595 (2010).
32. Depristo,M.A. et al. A framework for variation discovery and genotyping using next-generation DNA sequencing data. Nat. Genet 43, 491-498 (2011).
33. He,R. et al. Characterization of the inflammatory and apoptotic cells in the aortas of patients with ascending thoracic aortic aneurysms and dissections. J. Thorac. Cardiovasc. Surg. 131, 671-678 (2006).
34. Guo,D.C. & Milewicz,D.M. Methodology for using a universal primer to label amplified DNA segments for molecular analysis. Biotechnol. Lett. 25, 2079-2083 (2003).

## Claims

1. A method for determining whether a subject is predisposed to thoracic aortic aneurysms comprising detecting a *TGFB2* loss of function mutation wherein the presence of the mutation indicated that the subject is predisposed to thoracic aortic aneurysms.

2. The method according to claim 1 wherein the TGFB2 loss of function mutation is selected from the group consisting of the 5 base pair deletion c.1021_1025delTACAA in exon 6 of the gene, the nonsense p.Cys229* mutation, the nonsense p.Glu102* mutation or frameshift duplication (c.873_888dup) leading to p.Asn297.

3. A transforming growth factor beta-2 (TGF-β2) polypeptide for use in the treatment of a subject predisposed to thoracic aortic aneurysms, the thoracic aortic aneurysms being caused by a *TGFB2* loss of function mutation.

4. The polypeptide for its use according to claim 3, wherein the TGFB2 loss of function mutation is selected from the group consisting of the 5 base pair deletion c.1021_1025delTACAA in exon 6 of the gene, the nonsense p.Cys229* mutation, the nonsense p.Glu102* mutation or frameshift duplication (c.873_888dup) leading to p.Asn297.

## Patentansprüche

1. Verfahren zum Ermitteln, ob ein Proband eine Prädisposition für thorakale Aortenaneurysmen hat, umfassend das Detektieren einer TGFB2 - Funktionsverlustmutation, wobei das Vorhandensein der Mutation anzeigt, dass der Proband eine Prädisposition für thorakale Aortenaneurysmen hat.

2. Verfahren nach Anspruch 1, wobei die TGFB2 - Funktionsverlustmutation ausgewählt ist aus der Gruppe bestehend aus 5-Basenpaar-Verlust c.1021_1025delTACAA im Exon 6 des Gens, Nonsense-p.Cys229*-Mutation, Nonsense-p.Glu102*-Mutation oder Leseraster-Verschiebungs-Duplikation (c.873_888dup), die zu p.Asn297 führt.

3. Transformierendes Wachstumsfaktor-beta-2 (TGF-β2) Polypeptid zur Verwendung in der Behandlung eines Probanden mit einer Prädisposition für thorakale Aortenaneurysmen, wobei die thorakalen Aortenaneurysmen durch eine TGFB2 - Funktionsverlustmutation verursacht sind.

4. Polypeptid für seine Verwendung nach Anspruch 3, wobei die TGFB2 - Funktionsverlustmutation ausgewählt ist aus der Gruppe bestehend aus 5-Basenpaar-Verlust c.1021_1025delTACAA im Exon 6 des Gens, Nonsense-p.Cys229*-Mutation, Nonsense-p.Glu102*-Mutation oder Leseraster-Verschiebungs-Duplikation (c.873_888dup), die zu p.Asn297 führt.

## Revendications

1. Procédé pour déterminer si un sujet est prédisposé à des anévrismes aortiques thoraciques comprenant la détection d'une mutation de perte de fonction de *TGFB2,* où la présence de la mutation indique que le sujet est prédisposé à des anévrismes aortiques thoraciques.

2. Procédé selon la revendication 1, dans lequel la mutation de perte de fonction du TGFB2 est sélectionnée dans le groupe consistant en la délétion de 5 paires de bases c.1021_1025delTACAA dans l'exon 6 du gène, la mutation non-sens p.Cys229*, la mutation non-sens p.Glu102* ou une duplication avec décalage du cadre de lecture (c.873_888dup) donnant lieu à p.Asn297.

3. Polypeptide du facteur de croissance transformant bêta-2 (TGF-β2) destiné à être utilisé dans le traitement d'un sujet prédisposé à des anévrismes aortiques thoraciques, les anévrismes aortiques thoraciques étant provoqués par une mutation de perte de fonction de *TGFB2.*

4. Polypeptide destiné à être utilisé selon la revendication 3, où la mutation de perte de fonction du TGFB2 est sélectionnée dans le groupe consistant en la délétion de 5 paires de bases c.1021_1025delTACAA dans l'exon 6 du gène, la mutation non-sens p.Cys229*, la mutation non-sens p.Glu102* ou une duplication avec décalage du cadre de lecture (c.873_888dup) donnant lieu à p.Asn297.
